(19) **Europäisches Patentamt / European Patent Office / Office européen des brevets**

(11) **EP 2 303 110 B1**

(12) **EUROPEAN PATENT SPECIFICATION**

(45) Date of publication and mention of the grant of the patent:
**05.03.2014 Bulletin 2014/10**

(21) Application number: **09772910.7**

(22) Date of filing: **29.06.2009**

(51) Int Cl.:
*A61B 5/00* (2006.01)

(86) International application number:
**PCT/IB2009/006183**

(87) International publication number:
**WO 2010/001249 (07.01.2010 Gazette 2010/01)**

(54) **CONCATENATED SCALOGRAMS**

VERKNÜPFTE SKALOGRAMME

SCALOGRAMMES ENCHAÎNÉS

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **30.06.2008 US 77062 P**
**30.06.2008 US 77130 P**
**07.05.2009 US 437317**

(43) Date of publication of application:
**06.04.2011 Bulletin 2011/14**

(73) Proprietor: **Nellcor Puritan Bennett Ireland Mervue**
**Galway (IE)**

(72) Inventors:
• **McGONIGLE, Scott**
**Edinburgh EH11 1HR (GB)**
• **ADDISON, Paul, Stanley**
**Edinburgh EH10 6UR (GB)**

• **WATSON, James, Nicholas**
**Fife KY11 8LE (GB)**

(74) Representative: **Hargreaves, Timothy Edward et al**
**Marks & Clerk LLP**
**Atholl Exchange**
**6 Canning Street**
**Edinburgh EH3 8EG (GB)**

(56) References cited:
**US-A1- 2005 070 774    US-A1- 2006 258 921**

• **DAVID CLIFTON ET AL: "Measurement Of Respiratory Rate From the Photoplethysmogram In Chest Clinic Patients" JOURNAL OF CLINICAL MONITORING AND COMPUTING, KLUWER ACADEMIC PUBLISHERS, DO, vol. 21, no. 1, 25 November 2006 (2006-11-25), pages 55-61, XP019466386 ISSN: 1573-2614**

EP 2 303 110 B1

**Description**

**Cross Reference to Related Application**

This claims the benefit of United States Provisional Application No. 61/077,062 filed June 30, 2008, and United States Provisional Application No. 61/077,130, filed June 30, 2008.

**Summary**

The present disclosure relates to signal processing systems and methods, and more particularly, to systems and methods for concatenating selected regions of scalograms generated from an original signal. In an embodiment, the original signal or a portion thereof may be analyzed or reproduced in the creation of the concatenated scalogram.

For purposes of illustration, and not by way of limitation, in an embodiment disclosed herein the original signal is a photoplethysmograph (PPG) signal obtained from any suitable source, such as a pulse oximeter, and selected portions are the up and down stroke of a pulse (a pulse is a portion of the PPG signal corresponding to a heart beat), which are used to create separate new signals for further analysis. Further analysis includes determining respiration rate from the PPG signal using Secondary Wavelet Feature Decoupling (SWFD) applied to the new signals.

In an embodiment, the original signal may be selected and mirrored to create a new signal. The signal may be from any suitable source and may contain one or more repetitive components. In an embodiment, the selected signal is a portion of the original signal. The portion may be selected using any suitable method based on its characteristics, or characteristics of the original signal (*e.g.*, using local maximum and minimum values, or using second derivatives to find one or more turning points, of the original signal). By selecting a portion of the original signal and mirroring that portion, undesirable artifacts caused by the non-selected portion of the signal during further analysis may be removed and other benefits may be achieved. In an embodiment, additional portions of the original signal may be selected, mirrored, and added to the new signal. Alternatively, separate new signals may be created from the various mirrored portions.

In an embodiment, multiple up and down strokes are mirrored and combined to create new signals. The new signals are referred to herein as a "reconstructed up signal" for the series of pulses created from mirroring one or more up strokes selected from an original signal, or a "reconstructed down signal" for the series of pulses created from mirroring one or more down strokes selected from the original signal. In an embodiment, mirroring up and down strokes to create new signals may result in an improved analysis of the original PPG signal.

In an embodiment, a new signal may be generated by choosing characteristic points in the original signal or a scalogram generated from the original signal (e.g., points in the signal with local maxima or minima values) and interpolating between the values associated with the characteristic points. The resulting signal is referred to herein as an "interpolated signal." Unlike the mirroring technique discussed above, the temporal location of each point in the interpolated signal may be retained as compared to the original signal. This interpolated signal may be similar to the signal that results from mirroring a portion of the original signal to create a new signal as discussed above, or a signal extracted from the original signal (e.g., through a wavelet transform of this signal). The characteristic points that are chosen may correspond to the amplitude of an up and down stroke of a pulse (e.g., a portion of the signal corresponding to a heart beat). Interpolated signals that are created from characteristic points corresponding to upstroke amplitudes are referred to herein as an "interpolated up signal", and interpolated signals that are created from characteristic points corresponding to downstroke amplitudes are referred to herein as an "interpolated down signal". In an embodiment, interpolating between upstroke and downstroke amplitudes to create new signals may result in an improved analysis of the original PPG signal.

The signals selected for concatenation may be further analyzed using any suitable method, including for example (and as described herein for purposes of illustration), SWFD. In an embodiment of the disclosure, only one reconstructed or interpolated signal, instead of both reconstructed or interpolated signals, may be analyzed. A primary up scalogram and a primary down scalogram may be derived at least in part from the reconstructed up signal and down signal or interpolated up or down signal using any suitable method. For example, an up scalogram and the down scalogram may be derived using continuous wavelet transforms, including using a mother wavelet of any suitable characteristic frequency or form such as the Morlet wavelet with a particular scaling factor value. The up scalogram and the down scalogram also may be derived over any suitable range of scales. The resultant up scalogram and down scalogram may include ridges corresponding to at least one area of increased energy that may be analyzed further using any suitable method, for example using secondary wavelet feature decoupling.

The up ridge and the down ridge of the up and down scalograms may be extracted using any suitable method. For example, the up ridge and the down ridge may represent that at a particular scale value, the PPG signal may contain high amplitudes corresponding to the characteristic frequency of that scale. By extracting and further analyzing the ridges, information concerning the nature of the signal component associated with the underlying physical process causing a primary band on the up and down scalograms may also be extracted when the primary band itself is, for example, obscured in the presence of noise or other erroneous signal features. Secondary wavelet feature decoupling may be applied to each of the up and down ridges to derive secondary up and down scalograms. The secondary wavelet feature decoupling technique may provide desired information about the primary band by examining the amplitude modulation of a secondary band, such amplitude modulation being based at least in part on the presence of the signal component in the PPG signal that may be related to the primary band. This secondary wavelet decomposition of the up and down ridges allows for information concerning the band of interest to be made available as secondary bands for each of the secondary up and down scalograms. The secondary up and down scalograms may be derived using wavelets within a range of scales from any suitable minimum value up to any suitable maximum value and may be derived using any suitable scaling factor value for the wavelet. In an embodiment, secondary scalograms may be derived again at a lower scaling factor value so as to break up false ridges within the first set of secondary scalograms

In an embodiment, regions of the generated scalograms, for example the up and down scalograms , the secondary up and down scalograms, or the interpolated up and down scalograms discussed above, may be selected and concatenated. In an embodiment, regions of the original signals may be selected and concatenated. The regions chosen may be selected by a variety of methods. For example, the regions may be selected by consistency and / or stability in the scale and / or amplitude (e.g. energy) of ridges in the generated scalograms. In an embodiment, wavelet functions may be applied to the generated scalograms in order to further define ridges in the new signals. In addition, the regions may be selected based on characteristics of the original signals from which the scalograms were generated - for example, the peak and / or trough distance features of the original signals, localized scale of the signals, and / or the autocorrelation of the signals.

The selected regions of the original signal or scalograms generated from the original signal may be concatenated to form a concatenated scalogram. In an embodiment, the concatenated scalogram may include regions derived from both the up and down stroke of a pulse in the PPG signal. In an embodiment, the concatenated scalogram may include regions derived only from the up stroke of a pulse in the PPG signal, or only a down stroke in the PPG signal. In an embodiment, the concatenated scalogram may also contain regions derived from the raw signal scalogram, or may contain regions derived from scalograms of varying wavelet characteristics (e.g. higher or lower characteristic frequencies). In addition, the selected regions may be normalized and / or rescaled in scale and / or amplitude before, during, or after concatenation.

A sum along amplitudes across time may be applied to at least a portion of the concatenated scalogram to form a sum along amplitudes function. The sum along amplitudes may sum, for each scale increment within a range of scales, the amplitude (*e.g.*, the energy) or median amplitude of the concatenated scalogram. In an embodiment, outliers in scale and / or amplitude may be excluded from the sum along amplitudes calculation

A desired parameter may be determined based on the sum along amplitudes function. This determination may be made by identifying a characteristic point of the sum along amplitudes function. In an embodiment, a peak of the sum along amplitudes function may be analyzed to determine respiration information. In addition, areas of maximum curvature or gradient on the sum along amplitudes function may be analyzed to determine respiration information. In an embodiment, concatenating selected regions of scalograms that have been generated from original signals themselves may result in an improvement of the determination of respiration information. In an embodiment, concatenating selected regions of scalograms that have been generated from original signals in which portions of the original signals have been selected and mirrored may result in an improvement of the determination of respiration information. In an embodiment, concatenating selected regions of scalograms that have been generated from original signals in which portions of the original signals have been selected and interpolated, may result in an improvement of the determination of respiration information.

**Brief Description of the Drawings**

The above and other features of the present disclosure, its nature and various advantages will be more apparent upon consideration of the following detailed description, taken in conjunction with the accompanying drawings in which:

**FIG. 1** shows an illustrative pulse oximetry system in accordance with an embodiment;
**FIG. 2** is a block diagram of the illustrative pulse oximetry system of **FIG. 1** coupled to a patient in accordance with an embodiment;
**FIGS. 3(a)** and **3(b)** show illustrative views of a scalogram derived from a PPG signal in accordance with an embodiment;
**FIG. 3(c)** shows an illustrative scalogram derived from a signal containing two pertinent components in accordance with an embodiment;
**FIG. 3(d)** shows an illustrative schematic of signals associated with a ridge in **FIG. 3(c)** and illustrative schematics of a further wavelet decomposition of these newly derived signals in accordance with an embodiment;
**FIGS. 3(e)** and **3(f)** are flow charts of illustrative steps involved in performing an inverse continuous wavelet transform in accordance with embodiments;

**FIG. 4** is a block diagram of an illustrative continuous wavelet processing system in accordance with some embodiments;

**FIG. 5** is a flowchart of an illustrative process for selecting and mirroring portions of a signal to create a new signal for further analysis in accordance with an embodiment of the disclosure;

**FIG. 6** is a schematic of an illustrative process for reconstructing an up stroke signal and a down stroke signal from an original signal in accordance with an embodiment of the disclosure;

**FIG. 7** is a flowchart of an illustrative process for sampling and interpolating portions of a signal to create a new signal for further analysis in accordance with an embodiment of the disclosure;

**FIG. 8** is a schematic of an illustrative process for sampling and interpolating up stroke portions and down stroke portions of an original signal in accordance with an embodiment of the disclosure;

**FIG. 9** is a flowchart of an illustrative process for analyzing scalograms generated from an original signal using concatenated scalograms in accordance with an embodiment of the disclosure;

**FIG. 10** is a flowchart of an illustrative process for analyzing the reconstructed up stroke signal and down stroke signal of **FIG. 6** or the interpolated up signal and interpolated down signal of **FIG. 8** using concatenated scalograms in accordance with an embodiment of the disclosure;

**FIG. 11** is a schematic of an illustrative process for constructing a concatenated scalogram from scalograms created using the reconstructed up stroke signals and down stroke signal techniques in accordance with an embodiment of the disclosure.

## Detailed Description

An oximeter is a medical device that may determine the oxygen saturation of the blood. One common type of oximeter is a pulse oximeter, which may indirectly measure the oxygen saturation of a patient's blood (as opposed to measuring oxygen saturation directly by analyzing a blood sample taken from the patient) and changes in blood volume in the skin. Ancillary to the blood oxygen saturation measurement, pulse oximeters may also be used to measure the pulse rate of the patient. Pulse oximeters typically measure and display various blood flow characteristics including, but not limited to, the oxygen saturation of hemoglobin in arterial blood.

An oximeter may include a light sensor that is placed at a site on a patient, typically a fingertip, toe, forehead or earlobe, or in the case of a neonate, across a foot. The oximeter may pass light using a light source through blood perfused tissue and photoelectrically sense the absorption of light in the tissue. For example, the oximeter may measure the intensity of light that is received at the light sensor as a function of time. A signal representing light intensity versus time or a mathematical manipulation of this signal (*e.g.*, a scaled version thereof, a log taken thereof, a scaled version of a log taken thereof, etc.) may be referred to as the photoplethysmograph (PPG) signal. In addition, the term "PPG signal," as used herein, may also refer to an absorption signal (*i.e.*, representing the amount of light absorbed by the tissue) or any suitable mathematical manipulation thereof. The light intensity or the amount of light absorbed may then be used to calculate the amount of the blood constituent (*e.g.*, oxyhemoglobin) being measured as well as the pulse rate and when each individual pulse occurs.

The light passed through the tissue is selected to be of one or more wavelengths that are absorbed by the blood in an amount representative of the amount of the blood constituent present in the blood. The amount of light passed through the tissue varies in accordance with the changing amount of blood constituent in the tissue and the related light absorption. Red and infrared wavelengths may be used because it has been observed that highly oxygenated blood will absorb relatively less red light and more infrared light than blood with a lower oxygen saturation. By comparing the intensities of two wavelengths at different points in the pulse cycle, it is possible to estimate the blood oxygen saturation of hemoglobin in arterial blood.

When the measured blood parameter is the oxygen saturation of hemoglobin, a convenient starting point assumes a saturation calculation based on Lambert-Beer's law. The following notation will be used herein:

$$I(\lambda, t) = I_o(\lambda) \exp(-(s\beta_o(\lambda) + (1-s)\beta_r(\lambda))l(t)) \qquad (1)$$

where:

$\lambda$=wavelength;
t=time;
I=intensity of light detected;
$I_o$=intensity of light transmitted;

s=oxygen saturation;

$\beta_o$, $\beta_r$=empirically derived absorption coefficients; and

1(t)=a combination of concentration and path length from emitter to detector as a function of time.

The traditional approach measures light absorption at two wavelengths (*e.g.*, red and infrared (IR)), and then calculates saturation by solving for the "ratio of ratios" as follows.

1. First, the natural logarithm of (1) is taken ("log" will be used to represent the natural logarithm) for IR and Red

$$\log I = \log I_o - (s\beta_o + (1-s)\,\beta_r)l \qquad (2)$$

2. (2) is then differentiated with respect to time

$$\frac{d\log I}{dt} = -(s\beta_o + (1-s)\beta_r)\frac{dl}{dt} \qquad (3)$$

3. Red (3) is divided by IR (3)

$$\frac{d\log I(\lambda_R)/dt}{d\log I(\lambda_{IR})/dt} = \frac{s\beta_o(\lambda_R) + (1-s)\beta_r(\lambda_R)}{s\beta_o(\lambda_{IR}) + (1-s)\beta_r(\lambda_{IR})} \qquad (4)$$

4. Solving for s

$$s = \frac{\dfrac{d\log I(\lambda_{IR})}{dt}\beta_r(\lambda_R) - \dfrac{d\log I(\lambda_R)}{dt}\beta_r(\lambda_{IR})}{\dfrac{d\log I(\lambda_R)}{dt}(\beta_o(\lambda_{IR}) - \beta_r(\lambda_{IR})) - \dfrac{d\log I(\lambda_{IR})}{dt}(\beta_o(\lambda_R) - \beta_r(\lambda_R))}$$

Note in discrete time

$$\frac{d\log I(\lambda,t)}{dt} \simeq \log I(\lambda,t_2) - \log I(\lambda,t_1)$$

Using log A-log B=log A/B,

$$\frac{d\log I(\lambda,t)}{dt} \simeq \log\left(\frac{I(t_2,\lambda)}{I(t_1,\lambda)}\right)$$

So, (4) can be rewritten as

$$\frac{\dfrac{d\log I(\lambda_R)}{dt}}{\dfrac{d\log I(\lambda_{IR})}{dt}} \simeq \frac{\log\left(\dfrac{I(t_1,\lambda_R)}{I(t_2,\lambda_R)}\right)}{\log\left(\dfrac{I(t_1,\lambda_{IR})}{I(t_2,\lambda_{IR})}\right)} = R \qquad (5)$$

where **R** represents the "ratio of ratios." Solving (4) for s using (5) gives

$$s = \frac{\beta_r(\lambda_R) - R\beta_r(\lambda_{IR})}{R(\beta_o(\lambda_{IR}) - \beta_r(\lambda_{IR})) - \beta_o(\lambda_R) + \beta_r(\lambda_R)}.$$

From (5), **R** can be calculated using two points (*e.g.*, PPG maximum and minimum), or a family of points. One method using a family of points uses a modified version of (5). Using the relationship

$$\frac{d\log I}{dt} = \frac{dI/dt}{I} \qquad (6)$$

now (5) becomes

$$\frac{\dfrac{d\log I(\lambda_R)}{dt}}{\dfrac{d\log I(\lambda_{IR})}{dt}} \simeq \frac{\dfrac{I(t_2,\lambda_R) - I(t_1,\lambda_R)}{I(t_1,\lambda_R)}}{\dfrac{I(t_2,\lambda_{IR}) - I(t_1,\lambda_{IR})}{I(t_1,\lambda_{IR})}}$$

$$= \frac{[I(t_2,\lambda_R) - I(t_1,\lambda_R)]I(t_1,\lambda_{IR})}{[I(t_2,\lambda_{IR}) - I(t_1,\lambda_{IR})]I(t_1,\lambda_R)}$$

$$= R \qquad (7)$$

which defines a cluster of points whose slope of y versus x will give **R** where

$$x(t) = [I(t_2,\lambda_{IR}) - I(t_1,\lambda_{IR})]I(t_1,\lambda_R)$$
$$y(t) = [I(t_2,\lambda_R) - I(t_1,\lambda_R)]I(t_1,\lambda_{IR}) \qquad (8)$$
$$y(t) = Rx(t)$$

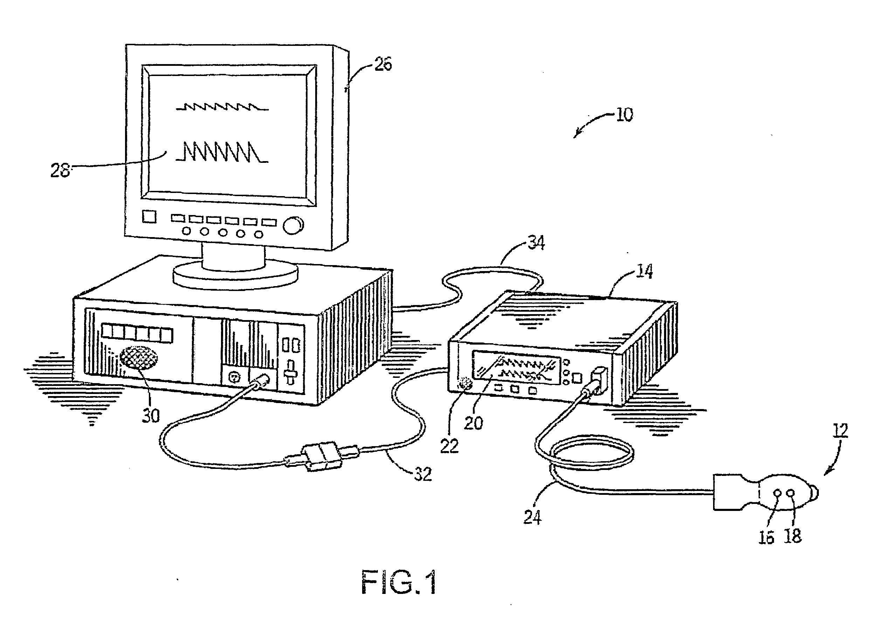

**FIG. 1** is a perspective view of an embodiment of a pulse oximetry system **10**. System **10** may include a sensor **12** and a pulse oximetry monitor **14**. Sensor **12** may include an emitter **16** for emitting light at two or more wavelengths into a patient's tissue. A detector **18** may also be provided in sensor **12** for detecting the light originally from emitter **16** that emanates from the patient's tissue after passing through the tissue.

According to another embodiment and as will be described, system **10** may include a plurality of sensors forming a sensor array in lieu of single sensor **12**. Each of the sensors of the sensor array may be a complementary metal oxide semiconductor (CMOS) sensor. Alternatively, each sensor of the array may be charged coupled device (CCD) sensor. In another embodiment, the sensor array may be made up of a combination of CMOS and CCD sensors. The CCD sensor may comprise a photoactive region and a transmission region for receiving and transmitting data whereas the CMOS sensor may be made up of an integrated circuit having an array of pixel sensors. Each pixel may have a photodetector and an active amplifier.

According to an embodiment, emitter **16** and detector **18** may be on opposite sides of a digit such as a finger or toe, in which case the light that is emanating from the tissue has passed completely through the digit. In an embodiment, emitter **16** and detector **18** may be arranged so that light from emitter **16** penetrates the tissue and is reflected by the tissue into detector **18,** such as a sensor designed to obtain pulse oximetry data from a patient's forehead.

In an embodiment, the sensor or sensor array may be connected to and draw its power from monitor **14** as shown. In another embodiment, the sensor may be wirelessly connected to monitor **14** and include its own battery or similar power supply (not shown). Monitor **14** may be configured to calculate physiological parameters based at least in part on data received from sensor **12** relating to light emission and detection. In an alternative embodiment, the calculations may be performed on the monitoring device itself and the result of the oximetry reading may be passed to monitor **14.** Further, monitor **14** may include a display **20** configured to display the physiological parameters or other information about the system. In the embodiment shown, monitor **14** may also include a speaker **22** to provide an audible sound that may be used in various other embodiments, such as for example, sounding an audible alarm in the event that a patient's physiological parameters are not within a predefined normal range.

In an embodiment, sensor **12,** or the sensor array, may be communicatively coupled to monitor **14** via a cable **24.** However, in other embodiments, a wireless transmission device (not shown) or the like may be used instead of or in addition to cable **24.**

In the illustrated embodiment, pulse oximetry system **10** may also include a multi-parameter patient monitor **26.** The monitor may be cathode ray tube type, a flat panel display (as shown) such as a liquid crystal display (LCD) or a plasma display, or any other type of monitor now known or later developed. Multi-parameter patient monitor **26** may be configured to calculate physiological parameters and to provide a display **28** for information from monitor **14** and from other medical monitoring devices or systems (not shown). For example, multiparameter patient monitor **26** may be configured to display an estimate of a patient's blood oxygen saturation generated by pulse oximetry monitor **14** (referred to as an "SpO$_2$" measurement), pulse rate information from monitor **14** and blood pressure from a blood pressure monitor (not shown) on display **28.**

Monitor **14** may be communicatively coupled to multi-parameter patient monitor **26** via a cable **32** or **34** that is coupled to a sensor input port or a digital communications port, respectively and/or may communicate wirelessly (not shown). In addition, monitor **14** and/or multi-parameter patient monitor **26** may be coupled to a network to enable the sharing of information with servers or other workstations (not shown). Monitor **14** may be powered by a battery (not shown) or by a conventional power source such as a wall outlet.

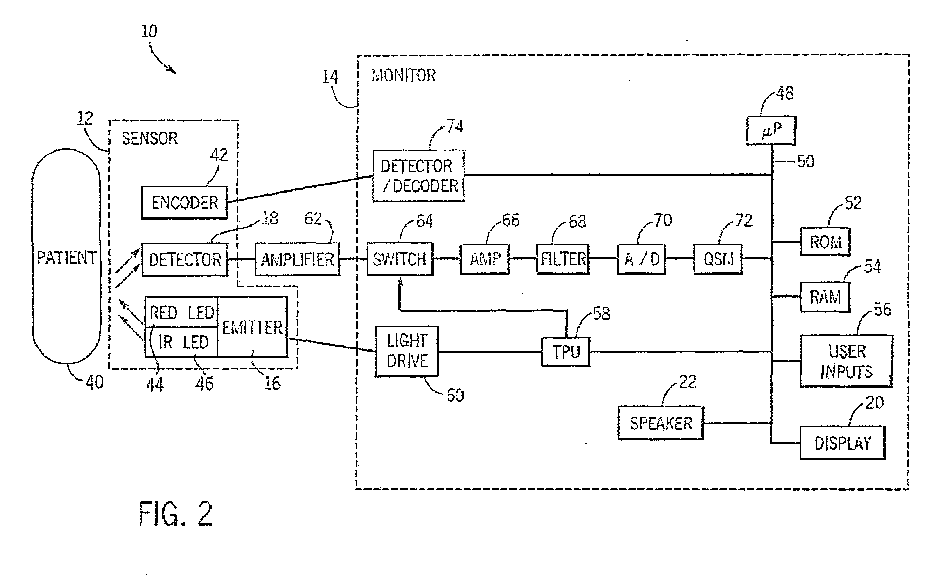

**FIG. 2** is a block diagram of a pulse oximetry system, such as pulse oximetry system **10** of **FIG. 1,** which may be coupled to a patient **40** in accordance with an embodiment. Certain illustrative components of sensor **12** and monitor **14** are illustrated in **FIG. 2.** Sensor **12 may** include emitter **16,** detector **18,** and encoder **42.** In the embodiment shown, emitter **16** may be configured to emit at least two wavelengths of light (*e.g., RED* and *IR*) into a patient's tissue **40.** Hence, emitter **16** may include a *RED* light emitting light source such as *RED* light emitting diode (LED) **44** and an *IR* light emitting light source such as *IR* LED **46** for emitting light into the patient's tissue **40** at the wavelengths used to calculate the patient's physiological parameters. In one embodiment, the *RED* wavelength may be between about 600 nm and about 700 nm, and the *IR* wavelength may be between about 800 nm and about 1000 nm. In embodiments where a sensor array is used in place of single sensor, each sensor may be configured to emit a single wavelength. For example, a first sensor emits only a *RED* light while a second only emits an *IR* light.

It will be understood that, as used herein, the term "light" may refer to energy produced by radiative sources and may include one or more of ultrasound, radio, microwave, millimeter wave, infrared, visible, ultraviolet, gamma ray or X-ray electromagnetic radiation. As used herein, light may also include any wavelength within the radio, microwave, infrared, visible, ultraviolet, or X-ray spectra, and that any suitable wavelength of electromagnetic radiation may be appropriate for use with the present techniques. Detector **18** may be chosen to be specifically sensitive to the chosen targeted energy spectrum of the emitter **16.**

In an embodiment, detector **18** may be configured to detect the intensity of light at the *RED* and *IR* wavelengths. Alternatively, each sensor in the array may be configured to detect an intensity of a single wavelength. In operation, light may enter detector **18** after passing through the patient's tissue **40.** Detector **18** may convert the intensity of the received light into an electrical signal. The light intensity is directly related to the absorbance and/or reflectance of light in the tissue **40.** That is, when more light at a certain wavelength is absorbed or reflected, less light of that wavelength is received from the tissue by the detector **18.** After converting the received light to an electrical signal, detector **18** may send the signal to monitor **14,** where physiological parameters may be calculated based on the absorption of the *RED* and *IR* wavelengths in the patient's tissue **40.**

In an embodiment, encoder **42** may contain information about sensor **12,** such as what type of sensor it is (*e.g.,* whether the sensor is intended for placement on a forehead or digit) and the wavelengths of light emitted by emitter **16.** This information may be used by monitor **14** to select appropriate algorithms, lookup tables and/or calibration coefficients stored in monitor **14** for calculating the patient's physiological parameters.

Encoder **42** may contain information specific to patient **40,** such as, for example, the patient's age, weight, and diagnosis. This information may allow monitor **14** to determine, for example, patient-specific threshold ranges in which the patient's physiological parameter measurements should fall and to enable or disable additional physiological parameter algorithms. Encoder **42** may, for instance, be a coded resistor which stores values corresponding to the type of sensor **12** or the type of each sensor in the sensor array, the wavelengths of light emitted by emitter **16** on each sensor of the sensor array, and/or the patient's characteristics. In another embodiment, encoder **42** may include a memory on which one or more of the following information may be stored for communication to monitor **14**: the type of the sensor **12**; the wavelengths of light emitted by emitter **16**; the particular wavelength each sensor in the sensor array is monitoring; a signal threshold for each sensor in the sensor array; any other suitable information; or any combination thereof.

In an embodiment, signals from detector **18** and encoder **42** may be transmitted to monitor **14**. In the embodiment shown, monitor **14** may include a general-purpose microprocessor **48** connected to an internal bus **50**. Microprocessor **48** may be adapted to execute software, which may include an operating system and one or more applications, as part of performing the functions described herein. Also connected to bus **50** may be a read-only memory (ROM) **52,** a random access memory (RAM) **54,** user inputs **56,** display **20,** and speaker **22**.

RAM **54** and ROM **52** are illustrated by way of example, and not limitation. Any suitable computer-readable media may be used in the system for data storage. Computer-readable media are capable of storing information that can be interpreted by microprocessor **48**. This information may be data or may take the form of computer-executable instructions, such as software applications, that cause the microprocessor to perform certain functions and/or computer-implemented methods. Depending on the embodiment, such computer-readable media may include computer storage media and communication media. Computer storage media may include volatile and non-volatile, removable and non-removable media implemented in any method or technology for storage of information such as computer-readable instructions, data structures, program modules or other data. Computer storage media may include, but is not limited to, RAM, ROM, EPROM, EEPROM, flash memory or other solid state memory technology, CD-ROM, DVD, or other optical storage, magnetic cassettes, magnetic tape, magnetic disk storage or other magnetic storage devices, or any other medium which can be used to store the desired information and which can be accessed by components of the system.

In the embodiment shown, a time processing unit (TPU) **58** may provide timing control signals to a light drive circuitry **60,** which may control when emitter **16** is illuminated and multiplexed timing for the *RED* LED **44** and the *IR* LED **46**. TPU **58** may also control the gating-in of signals from detector **18** through an amplifier **62** and a switching circuit **64**. These signals are sampled at the proper time, depending upon which light source is illuminated. The received signal from detector **18** may be passed through an amplifier **66,** a low pass filter **68,** and an analog-to-digital converter **70**. The digital data may then be stored in a queued serial module (QSM) **72** (or buffer) for later downloading to RAM **54** as QSM **72** fills up. In one embodiment, there may be multiple separate parallel paths having amplifier **66,** filter **68,** and A/D converter **70** for multiple light wavelengths or spectra received.

In an embodiment, microprocessor **48** may determine the patient's physiological parameters, such as $SpO_2$ and pulse rate, using various algorithms and/or look-up tables based on the value of the received signals and/or data corresponding to the light received by detector **18**. Signals corresponding to information about patient **40,** and particularly about the intensity of light emanating from a patient's tissue over time, may be transmitted from encoder **42** to a decoder **74**. These signals may include, for example, encoded information relating to patient characteristics. Decoder **74** may translate these signals to enable the microprocessor to determine the thresholds based on algorithms or loolc-up tables stored in ROM **52**. User inputs **56** may be used to enter information about the patient, such as age, weight, height, diagnosis, medications, treatments, and so forth. In an embodiment, display **20** may exhibit a list of values which may generally apply to the patient, such as, for example, age ranges or medication families, which the user may select using user inputs **56**.

The optical signal through the tissue can be degraded by noise, among other sources. One source of noise is ambient light that reaches the light detector. Another source of noise is electromagnetic coupling from other electronic instruments. Movement of the patient also introduces noise and affects the signal. For example, the contact between the detector and the skin, or the emitter and the skin, can be temporarily disrupted when movement causes either to move away from the skin. In addition, because blood is a fluid, it responds differently than the surrounding tissue to inertial effects, thus resulting in momentary changes in volume at the point to which the oximeter probe is attached.

Noise (*e.g.*, from patient movement) can degrade a pulse oximetry signal relied upon by a physician, without the physician's awareness. This is especially true if the monitoring of the patient is remote, the motion is too small to be observed, or the doctor is watching the instrument or other parts of the patient, and not the sensor site. Processing pulse oximetry (*i.e.*, PPG) signals may involve operations that reduce the amount of noise present in the signals or otherwise identify noise components in order to prevent them from affecting measurements of physiological parameters derived from the PPG signals.

It will be understood that the present disclosure is applicable to any suitable signals and that PPG signals are used merely for illustrative purposes. Those skilled in the art will recognize that the present disclosure has wide applicability to other signals including, but not limited to other biosignals (*e.g.*, electrocardiogram, electroencephalogram, electrogastrogram, electromyogram, heart rate signals, pathological sounds, ultrasound, or any other suitable biosignal), dynamic signals, non-destructive testing signals, condition monitoring signals, fluid signals, geophysical signals, astronomical signals, electrical signals, financial signals including financial indices, sound and speech signals, chemical signals, meteorological signals including climate signals, and/or any other suitable signal, and/or any combination thereof.

In one embodiment, a PPG signal may be transformed using a continuous wavelet transform. Information derived from the transform of the PPG signal (*i.e.*, in wavelet space) may be used to provide measurements of one or more physiological parameters.

The continuous wavelet transform of a signal x(t) in accordance with the present disclosure may be defined as

$$T(a,b) = \frac{1}{\sqrt{a}} \int_{-\infty}^{+\infty} x(t)\psi^* \left( \frac{t-b}{a} \right) dt \qquad (9)$$

where $\psi^*(t)$ is the complex conjugate of the wavelet function $\psi(t)$, *a* is the dilation parameter of the wavelet and *b* is the location parameter of the wavelet. The transform given by equation (9) may be used to construct a representation of a signal on a transform surface. The transform may be regarded as a time-scale representation. Wavelets are composed of a range of frequencies, one of which may be denoted as the characteristic frequency of the wavelet, where the characteristic frequency associated with the wavelet is inversely proportional to the scale *a.* One example of a characteristic frequency is the dominant frequency. Each scale of a particular wavelet may have a different characteristic frequency. The underlying mathematical detail required for the implementation within a time-scale can be found, for example, in Paul S. Addison, The Illustrated Wavelet Transform Handbook (Taylor & Francis Group 2002).

The continuous wavelet transform decomposes a signal using wavelets, which are generally highly localized in time. The continuous wavelet transform may provide a higher resolution relative to discrete transforms, thus providing the ability to garner more information from signals than typical frequency transforms such as Fourier transforms (or any other spectral techniques) or discrete wavelet transforms. Continuous wavelet transforms allow for the use of a range of wavelets with scales spanning the scales of interest of a signal such that small scale signal components correlate well with the smaller scale wavelets and thus manifest at high energies at smaller scales in the transform. Likewise, large scale signal components correlate well with the larger scale wavelets and thus manifest at high energies at larger scales in the transform. Thus, components at different scales may be separated and extracted in the wavelet transform domain. Moreover, the use of a continuous range of wavelets in scale and time position allows for a higher resolution transform than is possible relative to discrete techniques.

In addition, transforms and operations that convert a signal or any other type of data into a spectral (*i.e.*, frequency) domain necessarily create a series of frequency transform values in a two-dimensional coordinate system where the two dimensions may be frequency and, for example, amplitude. For example, any type of Fourier transform would generate such a two-dimensional spectrum. In contrast, wavelet transforms, such as continuous wavelet transforms, are required to be defined in a three-dimensional coordinate system and generate a surface with dimensions of time, scale and, for example, amplitude. Hence, operations performed in a spectral domain cannot be performed in the wavelet domain; instead the wavelet surface must be transformed into a spectrum (*i.e.*, by performing an inverse wavelet transform to convert the wavelet surface into the time domain and then performing a spectral transform from the time domain). Conversely, operations performed in the wavelet domain cannot be performed in the spectral domain; instead a spectrum must first be transformed into a wavelet surface (*i.e.*, by performing an inverse spectral transform to convert the spectral domain into the time domain and then performing a wavelet transform from the time domain). Nor does a cross-section of the three-dimensional wavelet surface along, for example, a particular point in time equate to a frequency spectrum upon which spectral-based techniques may be used. At least because wavelet space includes a time dimension, spectral techniques and wavelet techniques are not interchangeable. It will be understood that converting a system that relies on spectral domain processing to one that relies on wavelet space processing would require significant and fundamental modifications to the system in order to accommodate the wavelet space processing (*e.g.,* to derive a representative energy value for a signal or part of a signal requires integrating twice, across time and scale, in the wavelet domain while, conversely, one integration across frequency is required to derive a representative energy value from a spectral domain). As a further example, to reconstruct a temporal signal requires integrating twice, across time and scale, in the wavelet domain while, conversely, one integration across frequency is required to derive a temporal signal from a spectral domain. It is well known in the art that, in addition to or as an alternative to amplitude, parameters such as energy density, modulus, phase, among others may all be generated using such transforms and that these parameters have distinctly different contexts and meanings when defined in a two-dimensional frequency coordinate system rather than a three-dimensional wavelet coordinate system. For example, the phase of a Fourier system is calculated with respect to a single origin for all frequencies while the phase for a wavelet system is unfolded into two dimensions with respect to a wavelet's location (often in time) and scale.

The energy density function of the wavelet transform, the scalogram, is defined as

$$S(a,b) = \left| T(a,b) \right|^2 \qquad (10)$$

where '||' is the modulus operator. The scalogram may be rescaled for useful purposes. One common rescaling is defined as

$$S_R(a,b) = \frac{\left| T(a,b) \right|^2}{a} \qquad (11)$$

and is useful for defining ridges in wavelet space when, for example, the Morlet wavelet is used. Ridges are defined as the locus of points of local maxima in the plane. Any reasonable definition of a ridge may be employed in the method. Also included as a definition of a ridge herein are paths displaced from the locus of the local maxima. A ridge associated with only the locus of points of local maxima in the plane are labeled a "maxima ridge".

For implementations requiring fast numerical computation, the wavelet transform may be expressed as an approximation using Fourier transforms. Pursuant to the convolution theorem, because the wavelet transform is the cross-correlation of the signal with the wavelet function, the wavelet transform may be approximated in terms of an inverse FFT of the product of the Fourier transform of the signal and the Fourier transform of the wavelet for each required $a$ scale and then multiplying the result by $\sqrt{a}$ .

In the discussion of the technology which follows herein, the "scalogram" may be taken to include all suitable form of rescaling including, but not limited to, the original unscaled wavelet representation, linear rescaling, any power of the modulus of the wavelet transform, or any other suitable rescaling. In addition, for purposes of clarity and conciseness, the term "scalogram" shall be taken to mean the wavelet transform, T($a,b$) itself, or any part thereof. For example, the real part of the wavelet transform, the imaginary part of the wavelet transform, the phase of the wavelet transform, any other suitable part of the wavelet transform, or any combination thereof is intended to be conveyed by the term "scalogram".

A scale, which may be interpreted as a representative temporal period, may be converted to a characteristic frequency of the wavelet function. The characteristic frequency associated with a wavelet of arbitrary a scale is given by

$$f = \frac{f_c}{a} \qquad (12)$$

where $f_c$, the characteristic frequency of the mother wavelet (*i.e.*, at $a$=1), becomes a scaling constant and $f$ is the representative or characteristic frequency for the wavelet at arbitrary scale a.

Any suitable wavelet function may be used in connection with the present disclosure. One of the most commonly used complex wavelets, the Morlet wavelet, is defined as:

$$\psi(t) = \pi^{-1/4} (e^{i2\pi f_0 t} - e^{-(2\pi f_0)^2/2}) e^{-t^2/2} \qquad (13)$$

where $f_0$ is the central frequency of the mother wavelet. The second term in the parenthesis is known as the correction term, as it corrects for the non-zero mean of the complex sinusoid within the Gaussian window. In practice, it becomes negligible for values of $f_0 \gg 0$ and can be ignored, in which case, the Morlet wavelet can be written in a simpler form as

$$\psi(t) = \frac{1}{\pi^{1/4}} e^{i2\pi f_0 t} e^{-t^2/2} \qquad (14)$$

This wavelet is a complex wave within a scaled Gaussian envelope. While both definitions of the Morlet wavelet are included herein, the function of equation (14) is not strictly a wavelet as it has a non-zero mean (*i.e.,* the zero frequency term of its corresponding energy spectrum is non-zero). However, it will be recognized by those skilled in the art that equation (14) may be used in practice with $f_0$>>0 with minimal error and is included (as well as other similar near wavelet functions) in the definition of a wavelet herein. A more detailed overview of the underlying wavelet theory, including the definition of a wavelet function, can be found in the general literature. Discussed herein is how wavelet transform features may be extracted from the wavelet decomposition of signals. For example, wavelet decomposition of PPG signals may be used to provide clinically useful information within a medical device.

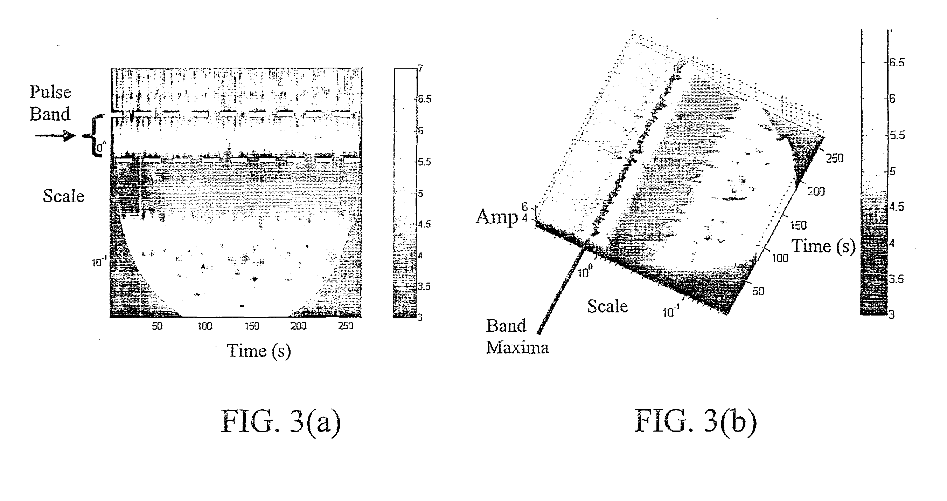

Pertinent repeating features in a signal give rise to a time-scale band in wavelet space or a rescaled wavelet space. For example, the pulse component of a PPG signal produces a dominant band in wavelet space at or around the pulse frequency. **FIGS. 3(a)** and **(b)** show two views of an illustrative scalogram derived from a PPG signal, according to an embodiment. The figures show an example of the band caused by the pulse component in such a signal. The pulse band is located between the dashed lines in the plot of **FIG. 3(a)**. The band is formed from a series of dominant coalescing features across the scalogram. This can be clearly seen as a raised band across the transform surface in **FIG. 3(b)** located within the region of scales indicated by the arrow in the plot (corresponding to 60 beats per minute). The maxima of this band with respect to scale is the ridge. The locus of the ridge is shown as a black curve on top of the band in **FIG. 3(b)**. By employing a suitable rescaling of the scalogram, such as that given in equation (11), the ridges found in wavelet space may be related to the instantaneous frequency of the signal. In this way, the pulse rate may be obtained from the PPG signal. Instead of rescaling the scalogram, a suitable predefined relationship between the scale obtained from the ridge on the wavelet surface and the actual pulse rate may also be used to determine the pulse rate.

By mapping the time-scale coordinates of the pulse ridge onto the wavelet phase information gained through the wavelet transform, individual pulses may be captured. In this way, both times between individual pulses and the timing of components within each pulse may be monitored and used to detect heart beat anomalies, measure arterial system compliance, or perform any other suitable calculations or diagnostics. Alternative definitions of a ridge may be employed. Alternative relationships between the ridge and the pulse frequency of occurrence may be employed.

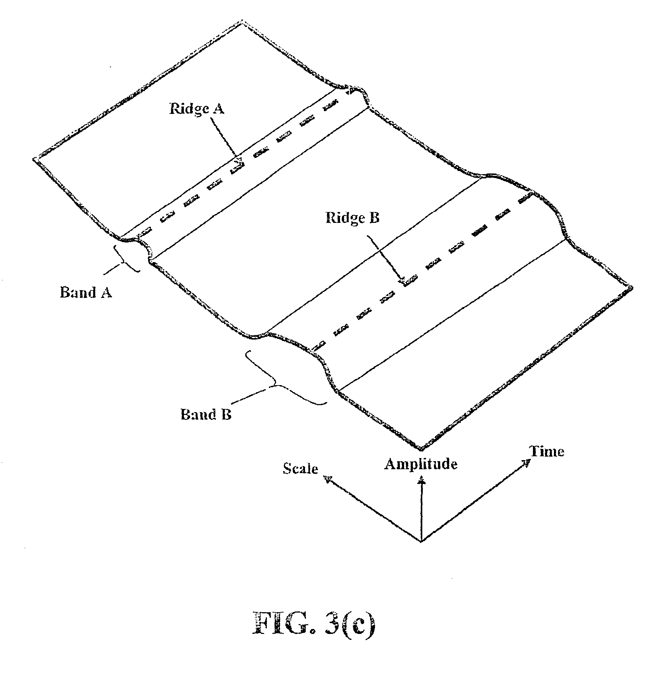

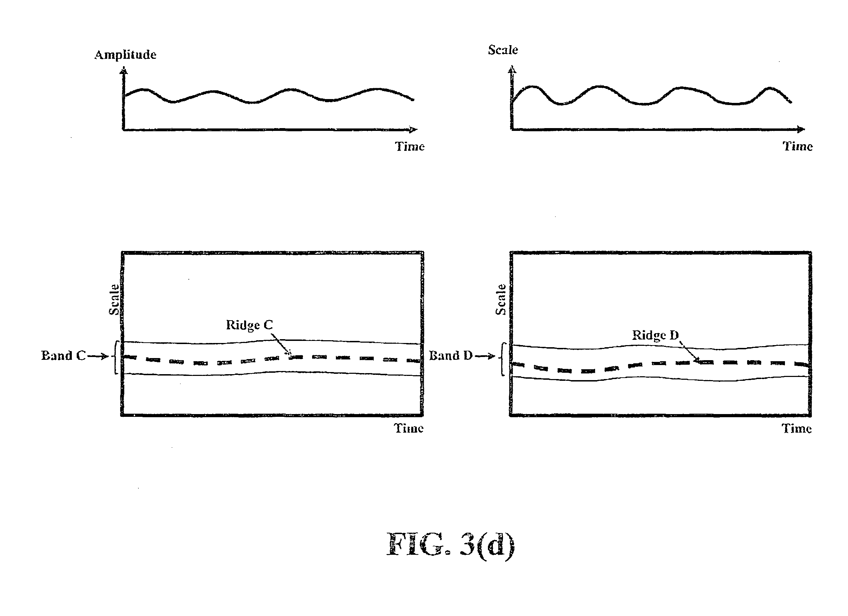

As discussed above, pertinent repeating features in the signal give rise to a time-scale band in wavelet space or a rescaled wavelet space. For a periodic signal, this band remains at a constant scale in the time-scale plane. For many real signals, especially biological signals, the band may be non-stationary; varying in scale, amplitude, or both over time. **FIG. 3(c)** shows an illustrative schematic of a wavelet transform of a signal containing two pertinent components leading to two bands in the transform space, according to an embodiment. These bands are labeled band A and band B on the three-dimensional schematic of the wavelet surface. In this embodiment, the band ridge is defined as the locus of the peak values of these bands with respect to scale. For purposes of discussion, it may be assumed that band B contains the signal information of interest. This will be referred to as the "primary band". In addition, it may be assumed that the system from which the signal originates, and from which the transform is subsequently derived, exhibits some form of coupling between the signal components in band A and band B. When noise or other erroneous features are present in the signal with similar spectral characteristics of the features of band B then the information within band B can become ambiguous (*i.e.*, obscured, fragmented or missing). In this case, the ridge of band A may be followed in wavelet space and extracted either as an amplitude signal or a scale signal which will be referred to as the "ridge amplitude perturbation" (RAP) signal and the "ridge scale perturbation" (RSP) signal, respectively. The RAP and RSP signals may be extracted by projecting the ridge onto the time-amplitude or time-scale planes, respectively. The top plots of **FIG. 3(d)** show a schematic of the RAP and RSP signals associated with ridge A in **FIG. 3(c)**. Below these RAP and RSP signals are schematics of a further wavelet decomposition of these newly derived signals. This secondary wavelet decomposition allows for information in the region of band B in **FIG. 3(c)** to be made available as band C and band D. The ridges of bands C and D may serve as instantaneous time-scale characteristic measures of the signal components causing bands C and D. This technique, which will be referred to herein as secondary wavelet feature decoupling (SWFD), may allow information concerning the nature of the signal components associated with the underlying physical process causing the primary band B **(FIG. 3(c))** to be extracted when band B itself is obscured in the presence of noise or other erroneous signal features.

In some instances, an inverse continuous wavelet transform may be desired, such as when modifications to a scalogram (or modifications to the coefficients of a transformed signal) have been made in order to, for example, remove artifacts. In one embodiment, there is an inverse continuous wavelet transform which allows the original signal to be recovered from its wavelet transform by integrating over all scales and locations, *a* and *b*:

$$x(t) = \frac{1}{C_g} \int_{-\infty}^{\infty} \int_{0}^{\infty} T(a,b) \frac{1}{\sqrt{a}} \psi\left(\frac{t-b}{a}\right) \frac{da\,db}{a^2} \qquad (15)$$

which may also be written as:

$$x(t) = \frac{1}{C_g} \int_{-\infty}^{\infty} \int_{0}^{\infty} T(a,b) \psi_{a,b}(t) \frac{\mathrm{d}a\mathrm{d}b}{a^2} \qquad (16)$$

where $C_g$ is a scalar value known as the admissibility constant. It is wavelet type dependent and may be calculated from:

$$C_g = \int_{0}^{\infty} \frac{|\hat{\psi}(f)|^2}{f} \, \mathrm{d}f \qquad (17)$$

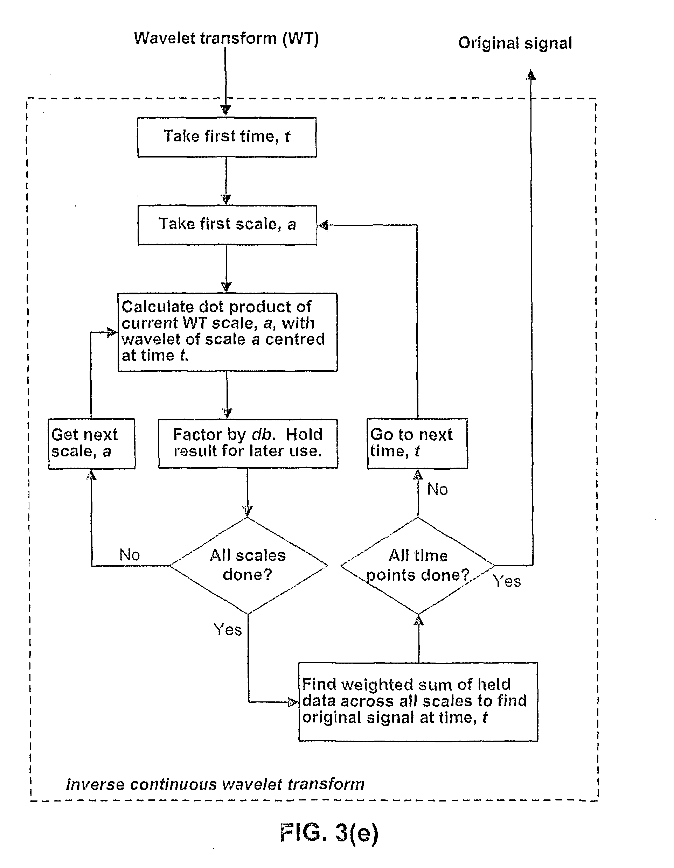

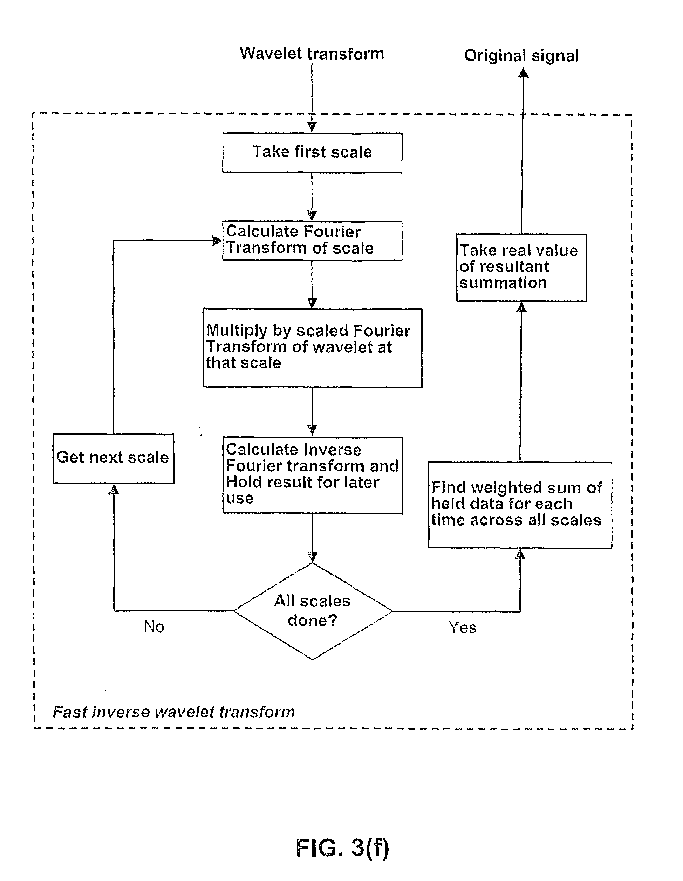

**FIG. 3(e)** is a flow chart of illustrative steps that may be taken to perform an inverse continuous wavelet transform in accordance with the above discussion. An approximation to the inverse transform may be made by considering equation (15) to be a series of convolutions across scales. It shall be understood that there is no complex conjugate here, unlike for the cross correlations of the forward transform. As well as integrating over all of *a* and *b* for each time *t*, this equation may also take advantage of the convolution theorem which allows the inverse wavelet transform to be executed using a series of multiplications. **FIG. 3(f)** is a flow chart of illustrative steps that may be taken to perform an approximation of an inverse continuous wavelet transform. It will be understood that any other suitable technique for performing an inverse continuous wavelet transform may be used in accordance with the present disclosure.

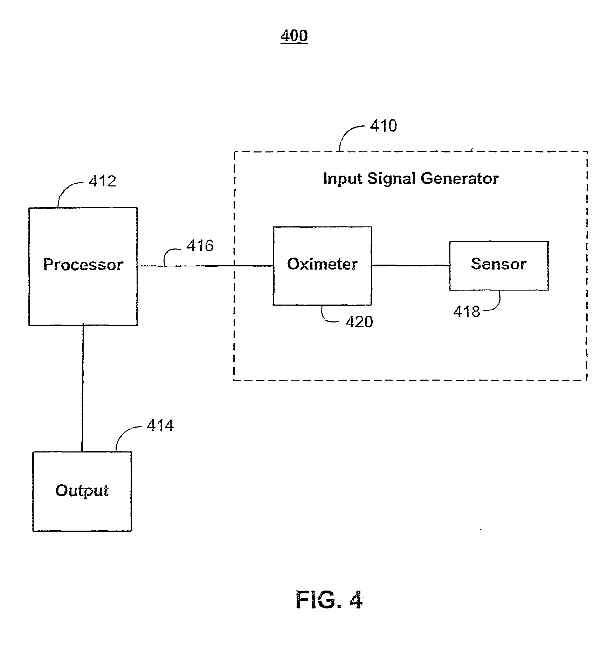

**FIG. 4** is an illustrative continuous wavelet processing system in accordance with an embodiment. In this embodiment, input signal generator **410** generates an input signal **416**. As illustrated, input signal generator **410** may include oximeter **420** coupled to sensor **418,** which may provide as input signal **416,** a PPG signal. It will be understood that input signal generator **410** may include any suitable signal source, signal generating data, signal generating equipment, or any combination thereof to produce signal **416.** Signal **416** may be any suitable signal or signals, such as, for example, biosignals (*e.g.*, electrocardiogram, electroencephalogram, electrogastrogram, electromyogram, heart rate signals, pathological sounds, ultrasound, or any other suitable biosignal), dynamic signals, non-destructive testing signals, condition monitoring signals, fluid signals, geophysical signals, astronomical signals, electrical signals, financial signals including financial indices, sound and speech signals, chemical signals, meteorological signals including climate signals, and/or any other suitable signal, and/or any combination thereof.

In this embodiment, signal **416** may be coupled to processor **412.** Processor **412** may be any suitable software, firmware, and/or hardware, and/or combinations thereof for processing signal **416.** For example, processor **412** may include one or more hardware processors (*e.g.*, integrated circuits), one or more software modules, computer-readable media such as memory, firmware, or any combination thereof. Processor **412** may, for example, be a computer or may be one or more chips (*i.e.*, integrated circuits). Processor **412** may perform the calculations associated with the continuous wavelet transforms of the present disclosure as well as the calculations associated with any suitable interrogations of the transforms. Processor **412** may perform any suitable signal processing of signal **416** to filter signal **416,** such as any suitable band-pass filtering, adaptive filtering, closed-loop filtering, and/or any other suitable filtering, and/or any combination thereof.

Processor **412** may be coupled to one or more memory devices (not shown) or incorporate one or more memory devices such as any suitable volatile memory device (*e.g.,* RAM, registers, *etc.*), non-volatile memory device (*e.g.,* ROM, EPROM, magnetic storage device, optical storage device, flash memory, *etc.*), or both. The memory may be used by processor **412** to, for example, store data corresponding to a continuous wavelet transform of input signal **416,** such as data representing a scalogram. In one embodiment, data representing a scalogram may be stored in RAM or memory internal to processor **412** as any suitable three-dimensional data structure such as a three-dimensional array that represents the scalogram as energy levels in a time-scale plane. Any other suitable data structure may be used to store data representing a scalogram.

Processor **412** may be coupled to output **414.** Output **414** may be any suitable output device such as, for example, one or more medical devices (e.g., a medical monitor that displays various physiological parameters, a medical alarm, or any other suitable medical device that either displays physiological parameters or uses the output of processor **412** as an input), one or more display devices (*e.g.*, monitor, PDA, mobile phone, any other suitable display device, or any combination thereof), one or more audio devices, one or more memory devices (*e.g.*, hard disk drive, flash memory, RAM, optical disk, any other suitable memory device, or any combination thereof), one or more printing devices, any other suitable output device, or any combination thereof.

It will be understood that system **400** may be incorporated into system **10** (**FIGS. 1** and **2**) in which, for example, input signal generator **410** may be implemented as parts of sensor **12** and monitor **14** and processor **412** may be implemented as part of monitor **14.**

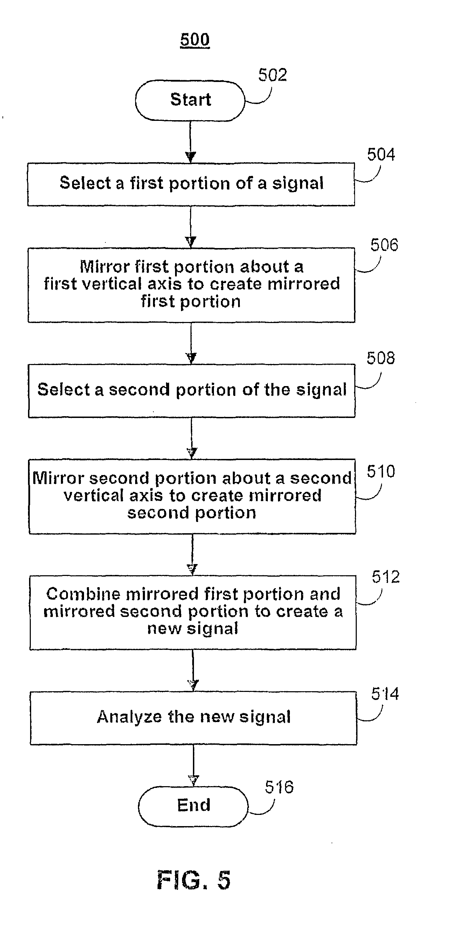

The continuous wavelet processing of the present disclosure will now be discussed in reference to **FIGS. 5-11.**

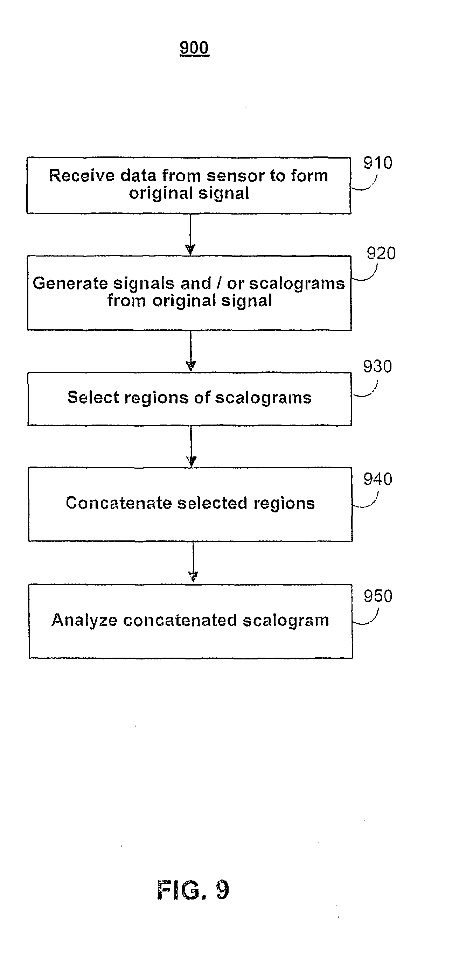

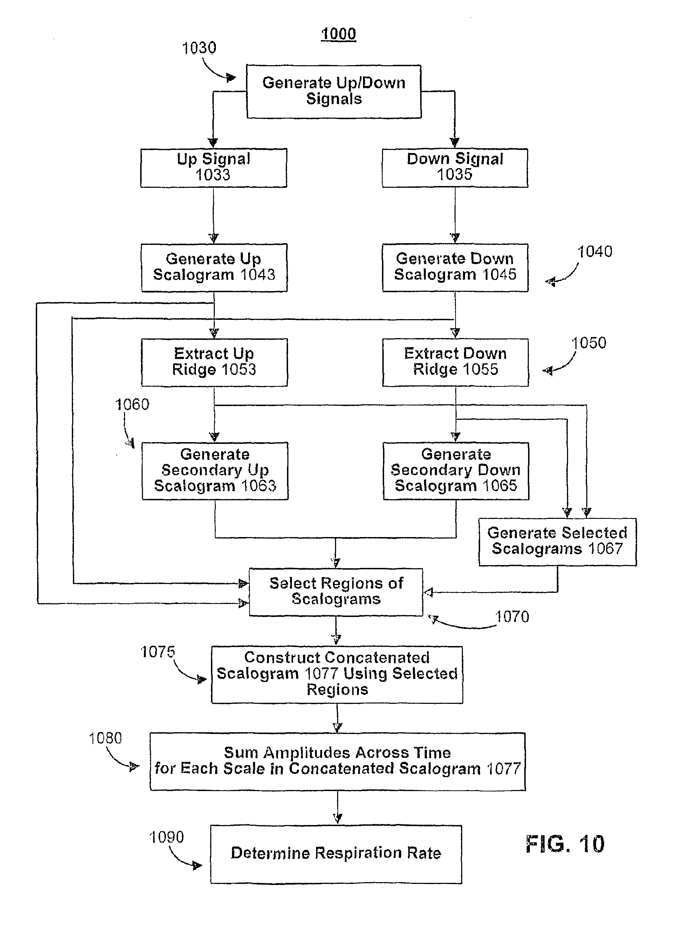

**FIG. 5** is a flowchart of an illustrative process for selecting and mirroring portions of a signal to create a new signal for further analysis in accordance with an embodiment of the disclosure. Process **500** may begin at step **502.** At step **504,** a first portion of an original signal may be selected. The original signal may include a signal from any suitable source and may contain one or more repetitive components. For example, the original signal may be a PPG signal. The first portion may be selected using any suitable method based on characteristics of the signal (*e.g.*, using local maximum and minimum values, or using second derivatives to find one or more turning points, of the original signal). The selected portion may correspond to a repetitive portion of the signal. For example, the selected portion may correspond to the up stroke or the down stroke of a PPG signal corresponding to a heartbeat. At step **506,** the first portion may be mirrored about any suitable first vertical axis to create a mirrored first portion such as a vertical axis located at the beginning or end of the selected segment. Process **500** may advance to step **508,** in which a second portion may be selected from the original signal. The second portion may be the same as, similar to, or different from the first portion, and may be selected using any suitable method. For example, the second portion may correspond to characteristics of the signal that occur subsequent in time to the first portion. At step **510,** the second portion of the original signal may be mirrored about any suitable second vertical axis to create a mirrored second portion. In an embodiment, process **500** may advance to step **512,** in which the mirrored first portion and the mirrored second portion may be combined to create a new signal. In an embodiment, process **500** may create two new signals: one from the mirrored first portion and one from the mirrored second portion. In this manner, one or more new signals may be created. These new signal may be analyzed further in step **514** using any suitable method, such any of the methods of process **900 (FIG. 9)** or process **1000 (FIG. 10)** discussed below. Process **500** may advance to step **516** and end.

The foregoing steps of the flowchart are merely illustrative and any suitable modifications may be made. For example, additional portions of the signal may be selected, mirrored, and added to the new signal. The process may be performed in real time as the signal is being received or may be performed after a signal has been received. The new signal may be analyzed using a wavelet transform such as a continuous wavelet transform.

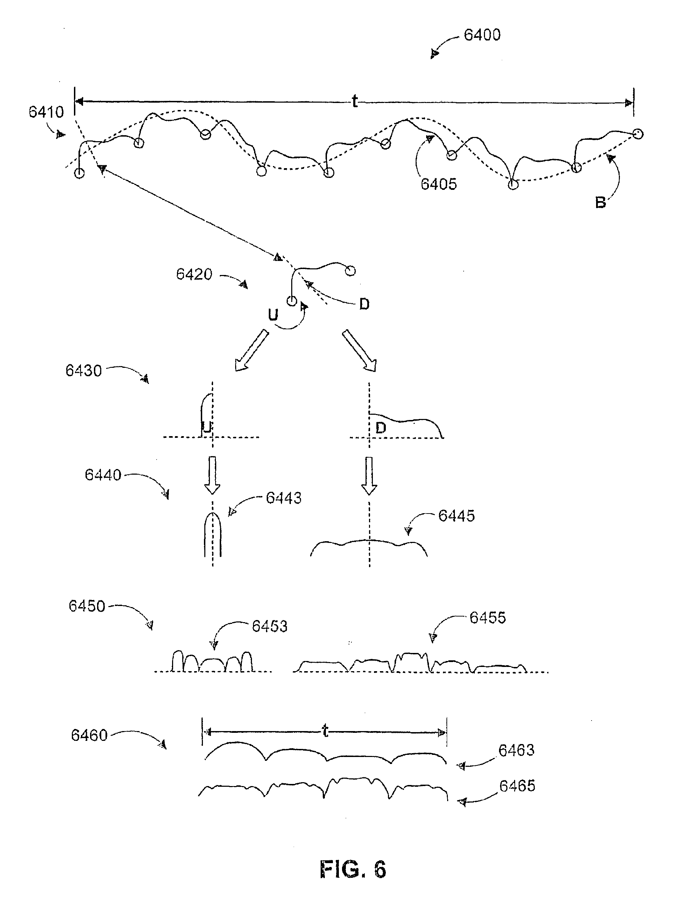

**FIG. 6** is a schematic of an illustrative process for reconstructing an up stroke signal and a down stroke signal from an original PPG signal in accordance with an embodiment of the disclosure. Process **6400** may be performed by processor **412 (FIG. 4)** or microprocessor **48 (FIG. 2)** in real time using a PPG signal obtained by sensor **12 (FIG. 2)** or input signal generator **410 (FIG. 4),** which may be coupled to patient **40,** using a time window smaller than the entire time window over which the PPG signal may be collected. Alternatively, process **6400** may be performed offline on PPG signal samples from QSM **72 (FIG. 2)** or from PPG signal samples stored in RAM **54** or ROM **52 (FIG. 2).,** using the entire time window of data over which the PPG signal was collected.

Process **6400** may begin at step **6410,** in which a PPG signal **6405** may be collected by sensor **12** or input signal generator **410** over any suitable time period t to reconstruct an up stroke signal **6463** and/or a down stroke signal **6465.** The portion of PPG signal **6405** used to reconstruct up signal **6463** and down signal **6465** may be selected using any suitable approach. For example, the up stroke and the down stroke of PPG signal **6405** may be selected based upon maximum and minimum values of PPG signal **6405.** Alternatively, a portion of PPG signal **6405** having an up stroke and a down stroke may be located using second derivatives to find one or more turning points of PPG signal **6405.** In an embodiment, processor **412** or microprocessor **48** may include any suitable software, firmware, and/or hardware, and/or combinations thereof for identifying maximum and minimum values of PPG signal **6405** and second derivatives of PPG signal **6405,** selecting a portion of PPG signal **6405,** and separating one or more up strokes in the portion of PPG signal **6405** from one or more down strokes. The local minimum turning points of PPG signal **6405** are shown in step **6410** using circles. In step **6420,** the up stroke and the down stroke may occur between two selected turning points, and the up stroke **"U"** may be distinguished from the down stroke **"D"** using a dotted line representing the local maximum value of PPG signal **6405** between and perpendicular to the two turning points of the original baseline **B** of PPG signal **6405.** In one suitable embodiment, the up stroke and the down stroke may be selected after filtering the PPG signal **6405** using, for example, a bandpass filter or low pass filter **68** to filter out frequencies higher and lower than the range of typical heart rates. In another suitable embodiment, the up and down strokes may be detected using techniques described in Watson, U.S. Provisional Application No. 61/077,092, filed June 30, 2008, entitled "Systems and Method for Detecting Pulses." Those skilled in the art will appreciate that any suitable method may be employed for the detection and/or selection of salient portions of the trace including but not limited to pattern matching methods (such as summation of differences or nearest neighbor techniques), syntactic processing methods (such as predicate calculus grammars), and adaptive methods (such as non-monotonic logic inference or artificial neural networks).

In **FIG. 6,** the original baseline **B** of PPG signal **6405** is shown as a sinusoidal-like dotted line, according to an embodiment. The baseline **B** may fluctuate due to the breathing of patient **40,** which may cause the PPG signal to oscillate, or twist, in the time plane. For example, PPG signal **6405** may experience amplitude modulation that may be related to dilation of the patient's vessels in correspondence with the patient's respiration. PPG signal **6405** may also include a carrier wave that may be based at least in part on the pressure in the patient's venous bed. PPG signal **6405** may also experience frequency modulation that may be based at least in part on a respiratory sinus arrhythmia of the patient. Process **6400** may remove the carrier wave of a PPG signal, the removal of which may be reflected at least in part in the amplitude modulation of the reconstructed up stroke signal and down stroke signal.

Process **6400** may advance to step **6420,** in which one up stroke and one down stroke of PPG signal **6405** may be selected by processor **412** or microprocessor **48** using any suitable method. In step **6420,** the up stroke and the down stroke may occur between two selected turning points, and the up stroke **"U"** may be distinguished from the down stroke **"D"** using a dotted line representing the local maximum value of PPG signal **6405** between and perpendicular to the two turning points. Any other suitable technique may be used to distinguish the up stroke and the down stroke. In an embodiment of the disclosure, up strokes of PPG signal **6405** may be selected for further processing by processor **412** or microprocessor **48** without also selecting down strokes from PPG signal **6405.** Similarly, down strokes of PPG signal **6405** may be selected for further processing without also selecting up strokes from PPG signal **6405.**

Process **6400** may advance to step **6430,** in which the up stroke selected at step **6420** may be separated from the selected down stroke by processor **412** or microprocessor **48** for further processing using any suitable method. For example, the up stroke may be separated from the down stroke at the point where the dotted line, representing the local maximum perpendicular to the two turning points, may intersect the selected portion of PPG signal **6405.**

Process **6400** may advance to step **6440,** in which each of the selected up stroke **"U"** and the selected down stroke **"D"** may be mirrored by processor **412** or microprocessor **48** about any suitable vertical axis. The shape of mirrored up pulse **6443** and mirrored down pulse **6445** may depend on which portion of PPG signal **6405** was selected at step **6420.** Because baseline **B** of PPG signal **6405** may fluctuate, an up stroke and down stroke combination selected from one portion of PPG signal **6405** may have a different amplitude and/or a different frequency than a similar up stroke and down stroke combination from another portion of PPG signal **6405.** For example, if in step **6420** a portion of PPG signal **6405** was selected in which the original baseline **B** was trending downwards, then the up stroke **"U"** and the resulting mirrored up signal may form a wider, flatter pulse while the down stroke **"D"** and the resulting mirrored down signal may form a narrower and taller pulse.

Process **6400** may advance to step **6450,** in which each of the mirrored up pulse **6443** and mirrored down pulse **6445** may be added to additional multiple pulses formed from the selection and mirroring of additional up strokes and down strokes from PPG signal **6405** to form mirrored up signal **6453** and mirrored down signal **6455.** Alternatively, mirrored up pulse **6443** and mirrored down pulse **6445** may each remain as an individual signal pulse and may be further analyzed by processor **412** or microprocessor **48** as described below with respect to **FIG. 9** and **FIG. 10.** Each of the pulses in mirrored up signal **6453** and mirrored down signal **6455** may vary in their amplitude and/or their time period, reflecting the amplitude and/or frequency oscillation of PPG signal **6405** in the time plane. Alternatively, each of the mirrored signals could be replicated to form a signal within a desired temporal window instead of forming a signal with a desired number of pulses.

Process **6400** may advance to step **6460,** in which each of mirrored up signal **6453** and mirrored down signal **6455** may be further manipulated by processor **412** or microprocessor **48** prior to further analysis, such as by being stretched or compressed to any desired size. Each pulse of the mirrored signals **6453** and **6455** may be expanded or shortened independently of the other pulses in the mirrored signals. For example, each of the pulses in the mirrored signals **6453** and **6455** may be stretched or compressed to make the time period for each pulse equal in size, where all of the time periods together equal the time period t over which PPG signal **6405** was collected or is being analyzed. Alternatively, each pulse of mirrored up signal **6453** and mirrored down signal **6455** may not be stretched to match time period t, but may instead be stretched or compressed to any desired size based at least in part on another time period of PPG signal **6405** or based at least in part on an individual or predetermined number of signal pulses. In an embodiment, each mirrored up pulse may be stretched or compressed to match the size of the up stroke used in the mirroring combined with its corresponding down stroke. The same process may be performed on each mirrored down pulse. In an embodiment, the mirrored pulses in mirrored signals **6453** and **6455** may be equally stretched or compressed to match the time period t over which the PPG signal **6405** was collected or is being analyzed.

The frequency modulation that occurs when one or more of the pulses in mirrored signals **6453** and **6455** is stretched or compressed may be converted into amplitude modulation by processor **412** or microprocessor **48** at step **6460** by increasing or decreasing the amplitude of each of the pulses in the mirrored signals **6453** and **6455** in relation to the amount of individual stretching or compressing described above. This may increase the amplitude modulation that may already exist in the mirrored pulses due to baseline changes in the original PPG signal **6405.** Translating the effect of the frequency modulation into amplitude modulation within the mirrored signals **6453** and **6455** may reduce the effect of respiratory sinus arrhythmia of patient **40** on further analysis of PPG signal **6405.** The amplitude of the pulses in reconstructed up signal **6463** and/or reconstructed down signal **6465** may be modulated or augmented if each of the pulses was stretched or compressed independently of each other (*e.g.*, to match the time period t over which PPG signal **6405** was collected and to match the period of each other pulse). Alternatively, the amplitude of each of the pulses in reconstructed up signal **6463** or reconstructed down signal **6465** may be the same (not shown) if the frequency modulation applied to the reconstructed signal stretched or compressed each pulse individually to create reconstructed signals with uniform amplitude. In an embodiment, reconstructed up signal **6463** and/or reconstructed down signal **6465** may include pulses that may vary in amplitude and frequency.

In an embodiment of the disclosure, an up stroke, but not a down stroke, may be selected in step **6420**, mirrored about a vertical axis in step **6440**, replicated in step **6450**, and stretched (or compressed) in step **6460**. Once the processing (e.g., selecting an up stroke and/or a down stroke, mirroring the strokes, replicating the mirrored pulses, and stretching or compressing the mirrored signals) of mirrored up signal **6453** and mirrored down signal **6455** is completed, then reconstructed up signal **6463** and reconstructed down stroke signal **6465** may be used in further processing by processor **412** or microprocessor **48** as described below with respect to **FIG. 9** and **10**.

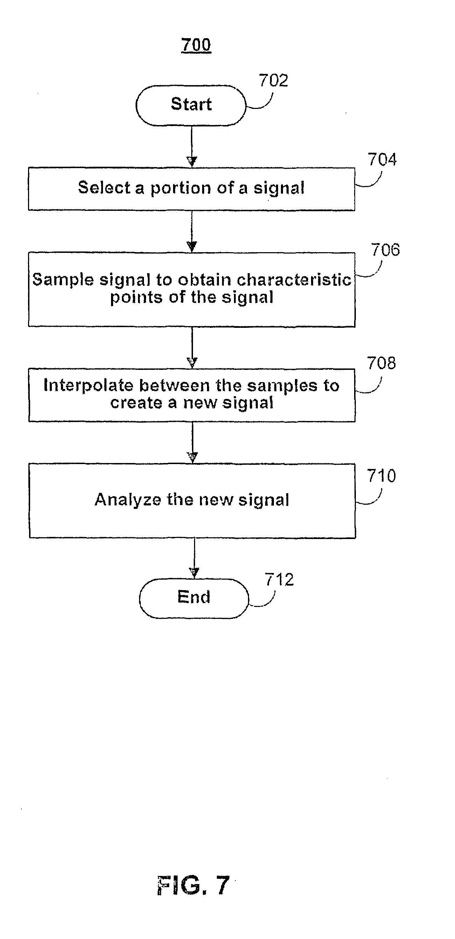

**FIG. 7** is a flowchart of an illustrative process for sampling and interpolating portions of a signal to create a new signal for further analysis in accordance with an embodiment of the disclosure. Process **700** may begin at step **702**. At step **704**, a portion of an original signal may be selected. The original signal may include a signal from any suitable source and may contain one or more repetitive components, as described with respect to step **504 (FIG. 5)**. For example, the selected portion may correspond to up strokes or down strokes of a PPG signal corresponding to a heart beat. Process **700** may then advance to step **706**. At step **706**, the portion of the original signal that was selected in step **704** may be sampled to obtain characteristic points of the signal. These samples may be taken at any particular frequency using any suitable characteristics of the selected portion of the original signal. Further, these samples may be taken using any suitable combination of amplifiers, filters, and / or analog-to-digital (A/D) converters, such as amplifier **66**, filter **68**, and A/D converter **70 (FIG. 2)**. The samples may then be stored in RAM **54** or ROM **52 (FIG. 2)** before being used for further processing. In an embodiment, points in the signal with local maxima or minima values may be sampled. For example, the characteristic points that are chosen may correspond to the amplitude of an up and down stroke of a pulse (e.g., a portion of the signal corresponding to a heart beat). Process **700** may then advance to step **708**.

At step **708**, interpolation may be performed using the characteristic points sampled at step **706** to create a new interpolated signal. This interpolation may be performed using any suitable methods known to those skilled in the art. For example, interpolation may be performed using curve fitting techniques such as a least squares approximation, a mean square error fit, polynomial interpolation, interpolation via a Gaussian process, or template matching. In an embodiment, process **700** may create two new signals: one using the characteristic points that correspond to the amplitude of an up stroke of a pulse (i.e., an interpolated up signal), and one created using the down stroke of a pulse (i.e., an interpolated down signal). In an embodiment, process **700** may create an interpolated signal that is a combination of characteristic points corresponding to both the up and down stroke of a pulse. Unlike the mirroring technique discussed with respect to processes **500** and **600 (FIG. 5** and **FIG. 6)**, the temporal location of each point in the interpolated signal may be retained as compared to the original signal. Further, the resulting interpolated signal may be similar to the signal that results from mirroring a portion of the original signal to create a new signal, as discussed with respect to processes **500** and **600** (**FIG. 5** and **FIG. 6**). The new interpolated signals created at step **708** may be analyzed further in step **710** using any suitable method, such as any of the methods of processes **900** and **1000** (**FIG. 9** and **FIG. 10**). Process **700** may advance to step **712** and end.

The foregoing steps of the flowchart are merely illustrative and any suitable modifications may be made. For example, additional portions of the signal may be selected and samples, and the samples may be interpolated to create signals that are added to the new signal. The process may be performed in real time as the signal is being received or may be performed after a signal has been received. The new signal may be analyzed using a wavelet transform such as a continuous wavelet transform.

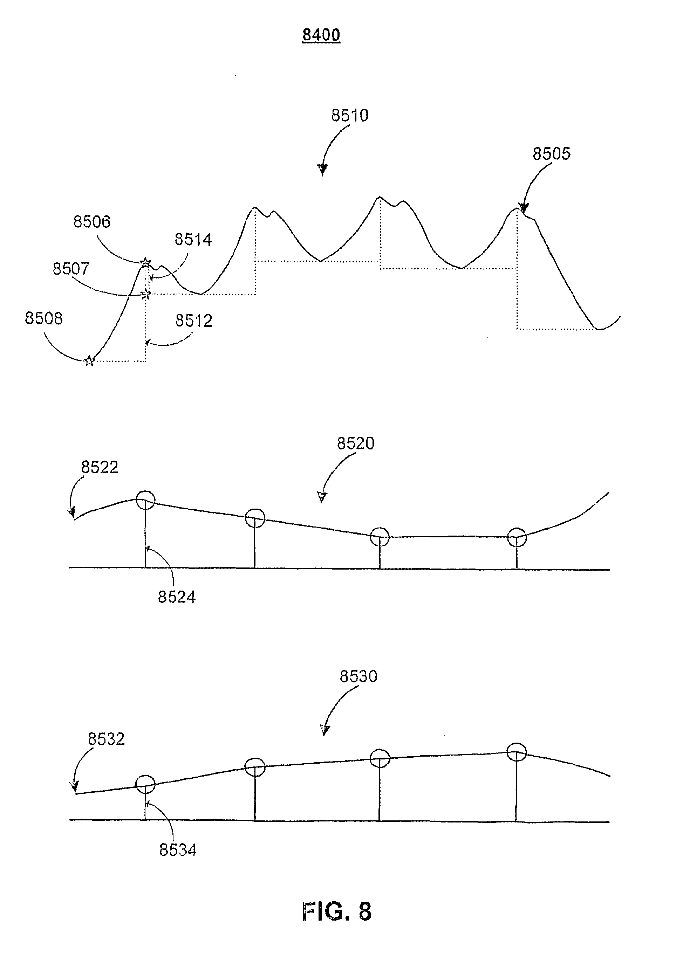

**FIG. 8** is a schematic of an illustrative process for sampling and interpolating up stroke portions and down stroke portions of an original signal in accordance with an embodiment of the disclosure. Process **8400** may be performed by processor **412 (FIG. 4)** or microprocessor **48 (FIG. 2)** in real time using a PPG signal obtained by sensor **12 (FIG. 2)** or input signal generator **410 (FIG. 4)**, which may be coupled to patient **40**, using a time window smaller than the entire time window over which the PPG signal may be collected. Alternatively, process **8400** may be performed offline on PPG signal samples from QSM **72 (FIG. 2)** or from PPG signal samples stored in RAM **54** or ROM **52 (FIG. 2)**., using the entire time window of data over which the PPG signal was collected.

Process **8400** may begin at step **8510**, in which a PPG signal **8505** may be collected by sensor **12** or input signal generator **410** over any suitable time period t to create an interpolated up signal **8522** and/or an interpolated down signal **8532**. A portion of the PPG signal **8505** may then be selected using any suitable approach. For example, the up strokes and down strokes of PPG signal **8505** may be selected based upon maximum and minimum values of PPG signal **8505** or second derivatives of PPG signal **8505**, as discussed with respect to step **6410** of process **6400** (**FIG. 6**). In an embodiment, processor **412** or microprocessor **48** may include any suitable software, firmware, and/or hardware, and/or combinations thereof to identify maximum and minimum values of PPG signal **8505**, selecting a portion of PPG signal **8505,** and separating one or more up strokes in the selected portion PPG signal **8505** from one or more down strokes. Like process **6400,** process **8400** may remove the carrier wave of a PPG signal, the removal of which may be reflected at least in part in the amplitude modulation of interpolated up signal **8522** and interpolated down signal **8532.**

At step **8510,** the portion of the original signal that was selected may be sampled to obtain characteristic points of the signal. These samples may be taken at any particular frequency using any suitable characteristics of the selected portion of the original signal. Further, these samples may be taken using any suitable combination of amplifiers, filters, and / or analog-to-digital (A/D) converters, such as amplifier **66,** filter **68,** and A/D converter **70 (FIG. 2).** The samples may then be stored in RAM **54** or ROM **52 (FIG. 2)** before being used for further processing. In an embodiment, the samples are chosen may correspond to the amplitude of an up and down stroke of a pulse. These up stroke and down stroke amplitudes may be calculated using local maximum and minimum values of PPG signal **8505** or second derivatives of PPG signal **8505.** For example, up stroke amplitude **8512** may be calculated as the difference between local maximum point **8506** and local minimum point **8508.** In addition, down stroke amplitude **8514** may be calculated as the difference between local maximum point **8506** and local minimum point **8507.** In an embodiment, the collected samples may be scaled, quantized, summed, or otherwise manipulated using any suitable techniques. Process **8400** may then advance to steps **8520** and **8530.**

At steps **8520** and **8530,** the samples calculated in step **8510** may be interpolated to create new signals. In an embodiment, the collected samples may be sorted into those that correspond to the amplitudes of up strokes in PPG signal **8505,** and those that correspond to the amplitudes of down strokes in PPG signal **8505.** For example, sample **8524** may correspond to up stroke amplitude **8512,** and may be grouped with other samples that correspond to the amplitudes of up strokes in PPG signal **8505.** In addition, sample **8534** may correspond to down stroke amplitude **8514,** and may be grouped with other samples that correspond to the amplitudes of down strokes in PPG signal **8505.** Interpolation may be performed on the samples using any suitable methods known to those skilled in the art. For example, interpolation may be performed using curve fitting techniques such as a least squares approximation, a mean square error fit, polynomial interpolation, interpolation via a Gaussian process, or template matching. In an embodiment, process **8400** may create two new signals. In step **8520,** an interpolated up signal may be created using samples that correspond to the amplitudes of up strokes in PPG signal **8505,** while at step **8530,** an interpolated down signal may be created using samples that correspond to the amplitudes of down strokes in PPG signal **8505.** In an embodiment, process **700** may create an interpolated signal that is a combination of samples corresponding to both the up and down strokes in PPG signal **8505.** Unlike the mirroring technique discussed with respect to processes **500** and **600 (FIG. 5** and **FIG. 6),** the temporal location of each point in the resulting interpolated signals may be retained as compared to the original signal. Further, the resulting interpolated signal may be similar to the signal that results from mirroring a portion of the original signal to create a new signal, as discussed with respect to processes **500** and **600 (FIG. 5** and **FIG. 6).** The new interpolated signals created at steps **8520** and **8530** may be used in further processing by processor **412** or microprocessor **48** as described below with respect to **FIG. 9** and **FIG. 10.**

**FIG. 9** is a flowchart of an illustrative process for analyzing scalograms generated from an original signal using concatenated scalograms in accordance with an embodiment of the disclosure. Process **900** may begin at step **910,** in which data is received from a sensor to form an original signal. For example, sensor **12 (FIG. 1)** may collect PPG signal in real time as the PPG signal is detected using sensor **12** or using input signal generator **410 (FIG. 4)** to form an original signal. Process **900** may then advance to step **920,** in which new signals are generated from the original signal. These new signals may be generated using any suitable signal processing techniques. In an embodiment, the new signals generated from the original signal may include the reconstructed up and down signals discussed with respect to **FIG. 5** and **FIG. 6.** In an embodiment, the new signals generated from the original signal may include interpolated up and down signals discussed with respect to **FIG. 7** and **FIG. 8.** In an embodiment, scalograms may be generated from these new signals. These scalograms may be generated using the same method (*e.g.,* using continuous wavelet transforms) that was used to derive the scalograms shown in **FIGS. 3(a), 3(b),** and **3(c).** In an embodiment, processor **412** or microprocessor **48** may perform the calculations associated with the continuous wavelet transforms of the new signals. The scalograms may be derived using a mother wavelet of any suitable characteristic frequency or form such as the Morlet wavelet where $f_o$ (which is related to its oscillatory nature) may take a value equal to 5.5 rads/sec, or any other suitable value. Process **900** may then advance to step **930.**

At step **930,** regions of the scalograms generated at step **920** may be may be analyzed by processor **412** or microprocessor **48** to select one or more desired regions, using any suitable method. For example, the scalograms may be analyzed to calculate regions above a threshold level of stability and / or consistency. Regions of stability and/or consistency may be selected, for example, using the techniques described in Watson et al., U.S. Application No. 12/437326, filed May 7, 2009, entitled "Consistent Signal Selection By Signal Segment Selection Techniques," (Attorney Docket Reference: H-RM-01424 COV-42). In an embodiment, wavelet functions may be applied to the scalograms before analyzing the scalograms. These wavelet functions may define ridges in the scalograms in wavelet space. For example, Morlet wavelets may be applied to the scalograms to define ridges in the scalograms in wavelet space. The ridges may then be extracted from the generated scalograms similar to the methods described with respect to step **1050**

**(FIG. 10).** In an embodiment, the regions may be selected according to characteristics of the scale and / or the amplitude of ridges in the scalograms. To analyze the ridges, a time window that may vary both in width and in start position (e.g., start time) may be slid across the one or more scalograms generated at step **920**. The ridges within the time window may be parameterized in terms of a weighting of the standard deviation of the path that the particular ridge fragment may take, in units of scale, the length of the ridge fragment, the proximity of the ridge to other ridges, and/or any other suitable weighting characteristics. The ridge having the highest weighting may be chosen for further processing by processor **412** or microprocessor **48**. In an embodiment, an area around the ridge having the highest weighting may be selected as a stable and / or consistent region within one of the generated scalograms.

In an embodiment, the regions of the generated scalograms may be analyzed and selected based on the original signals from which the scalograms were generated - e.g. the original signal formed at step **910**. For example, the peaks of the signals may be located. These peaks may then be analyzed to determine their consistency in amplitude in relation to other peaks in the signals, as described in Watson et al., U.S. Application No. 12/437326, filed May 7, 2009, entitled "Consistent Signal Selection By Signal Segment Selection Techniques," (Attorney Docket Reference: H-RM-01424 COV-42). In addition, the localized scale of the signal may be derived using a wavelet transform. The localized scale may then be analyzed to determine the troughs of the signals, or to determine the positions corresponding to the same relative phase of the signals. These positions may then be used to determine a select a stable region within a respective scalogram. In an embodiment, autocorrelations of the signals may be performed. These autocorrelations may then be used to select regions of a respective scalogram which give consistent indications of scale within the signal.

Process **900** may advance to step **940,** in which the regions of the scalograms selected in step **930** are concatenated. During concatenation, the selected regions of the scalograms may be scaled. For example, the frequency and / or the amplitude of the selected regions may be normalized during concatenation such that the resulting concatenated scalogram has a desired range of scale and / or amplitude, or particular maximum scale and / or amplitude. In an embodiment each region to be concatenated may be weighted and normalized by a confidence factor. In an embodiment, the selected regions may be concatenated without any further processing. The resulting concatenated scalogram may be represented in any suitable manner, such as plotting the selected regions of the scalograms in any suitable order in a single scalogram. Process **900** may then advance to step **950,** in which the concatenated scalogram may be used in further processing by processor **412** or microprocessor **48** as described below with respect to **FIG. 9** and **FIG. 10**.

**FIG. 10** is a flowchart of an illustrative process for analyzing the reconstructed up stroke signal and down stroke signal of **FIG. 6** or the interpolated up signal and interpolated down signal of **FIG. 8** using concatenated scalograms in accordance with an embodiment of the disclosure. Process **1000** may begin at step **1030,** in which up signal **1033** and down signal **1035,** which may be the same as, and may include some or all of the features of, reconstructed up signal **6463** and reconstructed down signal **6465** or interpolated up signal **8522** and interpolated down signal **8532,** respectively, may be generated from any original signal (*e.g.*, a PPG signal) using any suitable method. In an embodiment of the disclosure, only one reconstructed signal or interpolated signal (*e.g.*, up signal **1033**), instead of both reconstructed signals, may be analyzed by process **1000.**

Process **1000** may advance to step **1040,** in which a primary up scalogram **1043** and a primary down scalogram **1045** may be derived at least in part from up signal **1033** and down signal **1035** using any suitable method. For example, up scalogram **1043** and down scalogram **1045** may be derived using the same method (*e.g.*, using continuous wavelet transforms) that was used to derive the scalograms shown in **FIGS. 3(a), 3(b),** and **3(c).** In an embodiment, processor **412** or microprocessor **48** may perform the calculations associated with the continuous wavelet transforms of up signal **1033** and down signal **1035**. Up scalogram **1043** and down scalogram **1045** may be derived using a mother wavelet of any suitable characteristic frequency or form such as the Morlet wavelet where $f_o$ (which is related to its oscillatory nature) may take a value equal to 5.5 rads/sec, or any other suitable value.

Up scalogram **1043** and down scalogram **1045** also may be derived over any suitable range of scales. For example, up scalogram **1043** and down scalogram **1045** may be derived using wavelets within a range of scales whose characteristic frequencies span, for example, approximately 0.8 Hz on either side of the scale corresponding to band **A** as shown in **FIG. 3(c).** A narrower range of scales may be used to derive up scalogram **1043** and down scalogram **1045** to eliminate the inclusion of other artifacts (*e.g.*, noise), to focus on the component of interest within the PPG signal (*e.g.*, the pulse component), and to minimize the number of computations that processor **412** or microprocessor **48** would need to perform. The resultant up scalogram **1043** and down scalogram **1045** may include ridges corresponding to at least one area of increased energy, such as band **A** that may be analyzed further using any suitable method, for example using secondary wavelet feature decoupling.

Process **1000** may advance to step **1050,** in which an up ridge **1053** and a down ridge **1055** may be extracted by processor **412** or microprocessor **48** from up scalogram **1043** and down scalogram **1045,** respectively, using any suitable method. For example, up ridge **1053** and down ridge **1055** may represent that at a particular scale value, the PPG signal may contain high amplitudes corresponding to the characteristic frequency of that scale. The amplitude and/or scale modulation observed in band **A** may be the result of the effect of one component of the PPG signal (*e.g.*, a patient's respiration, as shown by breathing band **B** in **FIG. 3(c)**) on another component (*e.g.*, a patient's pulse rate, as shown by pulse band **A**). By extracting and further analyzing up ridge **1053** and/or down ridge **1055** with respect to band **A,** information concerning the nature of the signal component associated with the underlying physical process causing the primary band **B (FIG. 3(c))** may also be extracted when band **B** itself is, for example, obscured in the presence of noise or other erroneous signal features.

Process **1000** may advance to step **1060,** in which each of up ridge **1053** and down ridge **1055** may be transformed further into a secondary up scalogram **1063** and a secondary down scalogram **1065,** respectively, using any suitable method. In an embodiment, processor **412** or microprocessor **48** may perform the calculations associated with any suitable interrogations of the continuous wavelet transforms, including further transforming up ridge **1053** and down ridge **1055.** For example, secondary wavelet feature decoupling may be applied by processor **412** or microprocessor **48** to each of up ridge **1053** and down ridge **1055** to derive secondary up scalogram **763** and secondary down scalogram **765.** The secondary wavelet feature decoupling technique may provide desired information about the primary band **B** in **FIG. 3(c)** by examining the amplitude modulation of band **A,** such amplitude modulation being based at least in part on the presence of the signal component in the PPG signal that may be related to primary band **B.**

Up ridge **1053** or down ridge **1055** may be followed in wavelet space and extracted either as an amplitude signal (*e.g.,* the RAP signal as shown in **FIG. 3(d)**) and/or a scale signal (*e.g.,* the RSP signal as shown in **FIG. 3(d)**). In an embodiment, an "off-ridge" technique may be employed, in which a path near up ridge **1053** or down ridge **1055,** but not the maxima ridge itself, may be followed in wavelet space. The off-ridge technique may also be used to obtain amplitude modulation in the RAP signal.

The RAP and/or the RSP signal may be extracted by projecting up ridge **1053** or down ridge **1055** onto the time-amplitude plane. This secondary wavelet decomposition of up ridge **1053** and down ridge **1055** allows for information concerning the band of interest (*e.g.,* band **B** in **FIG. 3(c)**) to be made available as secondary bands (*e.g.,* band **C** and band **D** in **FIG. 3(d)**) for each of secondary up scalogram **1063** and secondary down scalogram **1065.** The ridges of the secondary bands may serve as instantaneous time-scale characteristic measures of the underlying signal components causing the secondary bands, which may be useful in analyzing the signal component associated with the underlying physical process causing the primary band of interest (*e.g.,* the breathing band **B**) when band **B** itself may be obscured.

In an embodiment, secondary up scalogram **1063** and secondary down scalogram **1065** may be derived by processor **412** or microprocessor **48** within a different window of scales than was used to derive up scalogram **1043** and down scalogram **1045.** Secondary up scalogram **1063** and secondary down scalogram **1065** may be derived using wavelets within a range of scales from any suitable minimum value, such as a scale whose characteristic frequency is approximately 0.07 Hz, up to any suitable maximum value, such as a scale at which the ridge of band **A** in **FIG. 3(c)** may be present. For example, using a window between a suitable minimum scale value and a scale value at which band **A** may be primarily located allows other signal components of the PPG signal (e.g., the breathing band represented by band **B**) to be analyzed. The window of scale values may still be chosen to eliminate the inclusion of other artifacts (e.g., noise) within the PPG signal.

Secondary up scalogram **1063** and secondary down scalogram **1065** may be derived by processor **412** or microprocessor **48** using any suitable value for scaling factor $f_c$ for the wavelet. For example, the value of $f_c$ may be lower than the value of $f_c$ used to derive up scalogram **743** and down scalogram **1045** to reduce the formation of continuous ridge paths in secondary up scalogram **1063** and secondary down scalogram **1065.** A lower value of $f_c$ may decrease the oscillatory nature of a wavelet.

Process **1000** may advance to step **1067,** which may be a repetition of step **1060** at a different value of $f_c$. The value of $f_c$ may be lower than the value used in step **1060** so as to break up false ridges within the scalograms of step **1067.** The ridge fragments formed within the repeated scalograms of step **1067** may be used to identify stable regions within secondary up scalogram **1063** and secondary down scalogram 1065.

Process **1000** may advance to step **1070,** in which regions of the scalograms generated in steps **1040, 1060,** and / or **1067** may be analyzed by processor **412** or microprocessor **48** to select one or more desired regions, using any suitable method. For example, any of up scalogram **1043,** down scalogram **1045,** secondary up scalogram **1063,** secondary down scalogram **1065,** and / or selected scalograms **1067** may be analyzed to calculate regions above a threshold level of stability and / or consistency. Regions of stability and / or consistency may be selected, for example, using the techniques described in Watson et al., U.S. Application No. 12/437326, filed May 7, 2009, entitled "Consistent Signal Selection By Signal Segment Selection Techniques," (Attorney Docket Reference: H-RM-01424 COV-42). In an embodiment, wavelet functions may be applied to the scalograms before analyzing the scalograms. These wavelet functions may define ridges in the scalograms in wavelet space. For example, Morlet wavelets may be applied to the scalograms to define ridges in the scalograms in wavelet space. The ridges may then be extracted from the generated scalograms similar to the methods described with respect to step **1050.** In an embodiment, the regions may be selected according to characteristics of the scale and / or the amplitude of ridges in the scalograms. To analyze the ridges, a time window that may vary both in width and in start position (e.g., start time) may be slid across the one or more up repeated scalograms and the one or more down repeated scalogram derived in each of steps **1060** and **1067.** The ridges within the time window may be parameterized in terms of a weighting of the standard deviation of the path that the particular ridge fragment may take, in units of scale, the length of the ridge fragment, the proximity of the ridge to other ridges, and/or any other suitable weighting characteristics. The ridge having the highest weighting may be chosen for further processing by processor **412** or microprocessor **48**. In an embodiment, an area around the ridge having the highest weighting may be selected as a stable and / or consistent region within one of the generated scalograms.

In an embodiment, the regions of the scalograms may be analyzed and selected based on the original signals from which the scalograms were generated - e.g. the signals from which the scalograms generated in steps **1040, 1060,** and / or **1067** originated. For example, the peaks of the signals may be located. These peaks may then be analyzed to determine their consistency in amplitude in relation to other peaks in the signals, as described in Watson et al., U.S. Application No. 12/437326, filed May 7, 2009, entitled "Consistent Signal Selection By Signal Segment Selection Techniques," (Attorney Docket Reference: H-RM-01424 COV-42). In addition, the localized scale of the signal may be derived using a wavelet transform. The localized scale may then be analyzed to determine the troughs of the signals, or to determine the positions corresponding to the same relative phase of the signals. These positions may then be used to determine a select a stable region within a respective scalogram. In an embodiment, autocorrelations of the signals may be performed. These autocorrelations may then be used to select regions of a respective scalogram which give consistent indications of scale within the signal.

Process **1000** may advance to step **1075,** in which a concatenated scalogram **1077** is constructed using the regions of the scalograms selected in step **1070**. For example, selected regions from secondary up scalogram **1063** and secondary down scalogram **1065** may be concatenated together to create concatenated scalogram **1077**. During concatenation, the selected regions of the scalograms may be scaled. For example, the frequency and / or the amplitude of the selected regions may be normalized during concatenation such that the resulting concatenated scalogram **1077** has a desired range of scale and / or amplitude, or particular maximum scale and / or amplitude. In an embodiment each region to be concatenated may be weighted and normalized by a confidence factor. In an embodiment, the selected regions may be concatenated without any further processing. The resulting concatenated scalogram **1077** may be represented in any suitable manner, such as plotting the selected regions of the scalograms in any suitable order in a single scalogram.

Process **1000** may advance to step **1080,** in which a sum along amplitudes across time technique may be applied by processor **412** or microprocessor **48** to concatenated scalogram **1077** constructed in step **1070** using any suitable method. In an embodiment, the sum along amplitudes technique may sum, for each scale increment within a range of scales, the amplitude (*e.g.*, the energy) of concatenated scalogram **1077** across a time window. In an embodiment, the sum along amplitudes technique may sum, for the median of the amplitudes for each scale increment within a range of scales, the median amplitudes of concatenated scalogram **1077**. The resulting sum may thereafter be represented in any suitable manner, such as by plotting the sum for each scale value as a function of scale value. In an embodiment, processor **412** or microprocessor **48** may include any suitable software, firmware, and/or hardware, and/or combinations thereof for generating a sum along amplitudes vector and applying it to concatenated scalogram **1077**. The sum along amplitudes technique may be applied to the entire concatenated scalogram **1077,** or only portions of concatenated scalogram **1077**. For example, the sum along amplitudes technique may not be applied to regions of concatenated scalogram **1077** that contain outliers. Regions of concatenated scalogram **1077** that include outliers may contain frequencies or amplitudes that are higher than the median frequency or amplitude of the signal by a multiple of the standard deviation of the frequencies or amplitudes in concatenated scalogram **1077.**

Process **1000** may then advance to step **1090,** in which the respiration rate of patient **40 (FIG. 1)** may be determined. The sum along amplitudes function calculated in step **1080** may be plotted as a function of scale value by processor **412** or microprocessor **48**. In an embodiment, the plot generated at step **1090** may be displayed in any suitable manner, including for example, on display **20 (FIG. 2),** display **28 (FIG. 2),** or output **414 (FIG. 4)** for review and analysis by a user of system **10 (FIG. 1)** or system **400 (FIG. 4).**

From the plot, a characteristic point may be chosen as the respiration rate of patient **40**. This characteristic point may be selected by processor **412,** microprocessor **48,** or by a user of system **10** or system **400**. In an embodiment, a peak of the sum along amplitudes function may be identified as the respiration rate of patient **40**. For example, the first peak or edge moving from a direction of decreasing scale along the sum along amplitudes function may be identified as the respiration rate of patient **40**. Alternatively, the maximal peak in the sum along amplitudes function may be identified as the respiration rate of patient **40**. In an embodiment, a point along the sum of amplitudes function other than a peak may be identified as the respiration rate of patient **40**. For example, a point corresponding to the area of maximum curvature or gradient of the sum along amplitudes function may be identified as the respiration rate of patient **40**.

Process **1000** may be applied to a PPG signal obtained from patient **40** in any suitable manner. In an embodiment, process **1000** may take the form of a computer algorithm that may be installed as part of system **10** or system **400**. The algorithm may be applied by processor **412** or microprocessor **48** to the PPG signal data in real time as the PPG signal is detected using sensor **12** or using input signal generator **410**. In an embodiment, the algorithm may be applied offline to PPG signal samples from QSM **72** or from PPG signal samples stored in RAM **54** or ROM **52**. The output of the algorithm, which may be displayed in any suitable manner (*e.g.*, using display **20,** display **28 ,** or output **414**) may include the respiration rate of patient **40,** which may be used by a user of system **10** or system **400** for any suitable purpose (*e.g.*, assessing the respiratory health of patient **40**). In an embodiment, the algorithm may provide several benefits in calculating the respiration rate of patient **40,** including for example, a significant decrease (*e.g.*, on the order of 400%) in the amount of time required to load the firmware associated with the algorithm onto system **10** or system **400.** The process **1000** algorithm may also significantly improve the number of samples, or the percentage of patient data, that may be used to determine the patient's respiration rate.

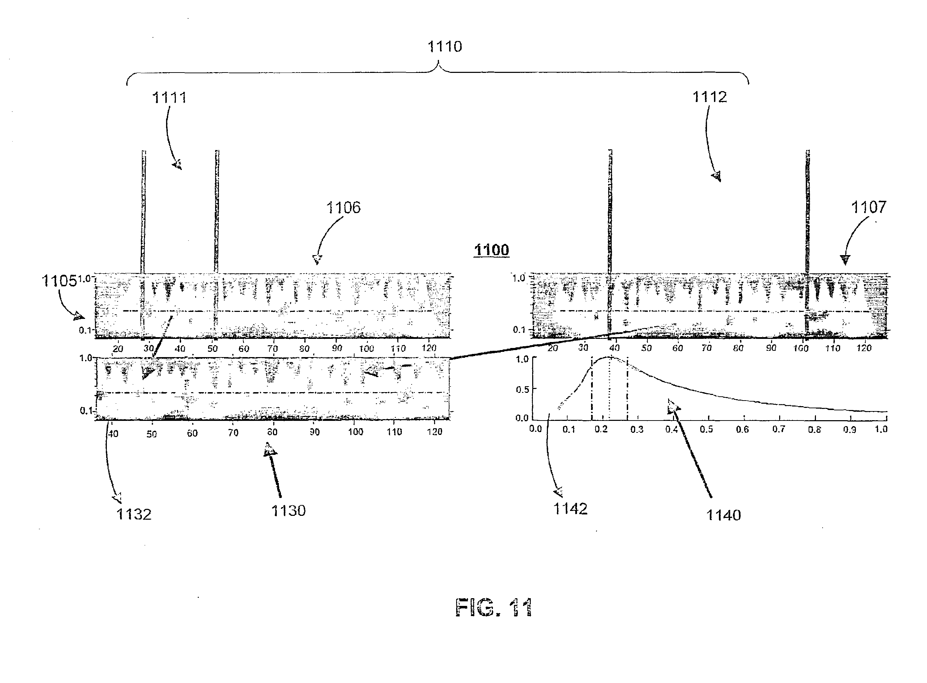

FIG. 11 is a schematic of an illustrative process **1100** for constructing a concatenated scalogram from scalograms created using the reconstructed up stroke signals and down stroke signal techniques in accordance with an embodiment of the disclosure. Process **1100** may be performed by processor **412 (FIG. 4)** or microprocessor **48 (FIG. 2)** in real time using a PPG signal obtained by sensor **12 (FIG. 2)** or input signal generator **410 (FIG. 4),** which may be coupled to patient **40,** using a time window smaller than the entire time window over which the PPG signal may be collected. Alternatively, process **1100** may be performed offline on PPG signal samples from QSM **72 (FIG. 2)** or from PPG signal samples stored in RAM **54** or ROM **52 (FIG. 2),** using the entire time window of data over which the PPG signal was collected.

Process **1100** may begin at step **1105,** in which scalograms are calculated and plotted according to any suitable method, such as process **6400 (FIG. 6),** process **8400 (FIG. 8)** and process **1000 (FIG. 10).** For example, at step **1105,** secondary up scalogram **1106** and secondary down scalogram **1107** are calculated from a PPG signal collected by sensor **12** or input signal generator **412** using step **1060** of process **1000,** and then plotted according to their respective scale and amplitude (e.g., energy) over time. In an embodiment, the plot generated at step **1105** may be displayed in any suitable manner, including for example, on display **20 (FIG. 2),** display **28 (FIG. 2),** or output **414 (FIG. 4)** for review and analysis by a user of system **10 (FIG. 1)** or system **400 (FIG. 4).**

Process **1100** may advance to step **1110,** in which the regions of scalograms **1105** are selected according to any suitable method, such as the methods described with respect to step **1070** of process **1000.** For example, at step **1110,** secondary up scalogram **1106** and secondary down scalogram **1107** are analyzed to determine which region of each respective scalogram is most stable, and region **1110** of secondary up scalogram **1106** and region **1120** of secondary down scalogram **1107** are selected.

Process **1100** may advance to step **1130,** in which a concatenated scalogram is constructed using the regions of the scalograms selected in step **1110.** Step **1130** may be performed substantially similarly to step **1075** of process **1000.** For example, at step **1130** region **1110** of secondary up scalogram **1106** and region **1120** of secondary down scalogram may be concatenated to form concatenated scalogram **1132.** In an embodiment, concatenated scalogram **832** may be displayed in any suitable manner, including for example, on display **20 (FIG. 2),** display **28 (FIG. 2),** or output **414 (FIG. 4)** for review and analysis by a user of system **10 (FIG. 1)** or system **400 (FIG. 4).**

Process **1100** may advance to step **1140,** in which sum along amplitudes techniques may be applied to concatenated scalogram **1132** constructed in step **1130** using any suitable method. Step **1140** may be performed substantially similarly to step **1080** of process **1000.** For example, at step **1140,** two different sum of amplitude functions may be applied to concatenated scalogram **1132,** and be plotted as graph **1142.** A first sum along amplitudes technique may sum, for each scale increment within a range of scales, the amplitude (e.g., the energy) of concatenated scalogram **1132** across a time window, and be plotted as a function of energy over scale value as the red line in plot **1142.** A second sum along amplitudes technique may sum, for the median of the amplitudes for each scale increment within a range of scales, the median amplitudes of concatenated scalogram **1132,** and be plotted as a function of energy over scale value as the blue line in plot **1142.** In an embodiment, characteristic points may be chosen from the calculated sum along amplitude functions to determine the respiration rate of patient **40**. The selection of characteristic points may be performed substantially similarly to step **1080** of process **1000.**

**Claims**

**1.** A signal processing method comprising:

    receiving data indicative of an original signal at a sensor;
    generating scalograms from the original signal;
    selecting regions of the generated scalograms based at least in part on at least one characteristic of the generated scalograms; **characterized by**
    concatenating the selected regions to form a concatenated scalogram;
    applying a sum along amplitudes across time to at least a portion of the concatenated scalogram to form a sum along amplitudes function; and
    determining a desired parameter based at least in part on the sum along amplitudes function.

**2.** The method of claim 1, wherein generating scalograms comprises generating a plurality of up scalograms and a plurality of down scalograms by:

selecting a first portion of the original signal;
mirroring the first portion of the original signal about a first vertical axis to create a mirrored first portion;
selecting a subsequent second portion of the original signal;
mirroring the second portion of the original signal about a second vertical axis to create a mirrored second portion;
combining the mirrored first portion and the mirrored second portion to create a new signal;
transforming the new signal using a wavelet transform; and
generating a scalogram based at least in part on the transformed signal, wherein
optionally the selected regions comprise at least one region of the up scalograms and at least one region of the down scalograms

**3.** The method of claim 1, wherein generating scalograms comprises generating a plurality of interpolated scalograms by:

selecting a portion of the original signal;
generating samples of the selected portion of the original signal using at least one characteristic of the original signal;
interpolating between the samples to create an interpolated signal;
transforming the interpolated signal using a wavelet transform; and
generating a scalogram based at least in part on the transformed signal, wherein
optionally the at least one characteristic comprises an amplitude of at least one of an up stroke and a down stroke of a pulse in the original signal.

**4.** The method of claim 1, wherein:-

the at least one characteristic comprises a peak in the original signal; or
the method further comprises selecting at least one ridge in at least one of the generated scalograms, wherein the at least one characteristic comprises consistency in at least one of the scale and amplitude of at least one ridge; or
concatenating the selected regions further comprise normalizing at least one of the scale and amplitude of the selected regions; or
applying a sum along amplitudes across time further comprises summing the median amplitude for each scale increment in the concatenated scalogram.

**5.** The method of claim 1, wherein applying a sum along amplitudes across time further comprises:

identifying at least one outlier in the concatenated scalogram; and
applying a sum along amplitudes across time to regions of the concatenated scalogram that do not contain the at least one outlier.

**6.** The method of claim 1, wherein determining the desired parameter further comprises:

selecting a peak of the sum along amplitudes function; and
analyzing the peak to obtain respiration information.

**7.** The method of claim 1, wherein determining the desired parameter further comprises:

identifying a point of maximum curvature of the sum along amplitudes function; and
analyzing the point to obtain respiration information.

**8.** A system for processing a signal, the system comprising:

a sensor for receiving data indicative of an original signal;
a processor coupled to the sensor, wherein the processor is configured to:

generate scalograms from the original signal;

select regions of the generated scalograms based at least in part on at least one characteristic of the generated scalograms; **characterized in that** the processor is further configured to

concatenate the selected regions to form a concatenated scalogram;

apply a sum along amplitudes across time to at least a portion of the concatenated scalogram to form a sum along amplitudes function;

determine a desired parameter based at least in part on the sum along amplitudes function; and

an output coupled to the processor, wherein the output is configured to display at least one of the concatenated scalogram, the sum along amplitudes function, and the determined parameter.

9. The system of claim 8, wherein the processor is further configured to:

select a first portion of the original signal;

mirror the first portion of the original signal about a first vertical axis to create a mirrored first portion;

select a subsequent second portion of the original signal;

mirror the second portion of the original signal about a second vertical axis to create a mirrored second portion;

combine the mirrored first portion and the mirrored second portion to create a new signal;

transform the new signal using a wavelet transform; and

generate a scalogram based at least in part on the transformed signal.

10. The system of claim 8, wherein the processor is further configured to:

select a portion of the original signal;

generate samples of the selected portion of the original signal using at least one characteristic of the original signal;

interpolate between the samples to create an interpolated signal;

transform the interpolated signal using a wavelet transform; and

generate a scalogram based at least in part on the transformed signal.

11. The system of claim 8, wherein:-

the at least one characteristic comprises an amplitude of at least one of an up stroke and a down stroke of a pulse in the original signal; or

the selected regions comprise at least one region of the up scalograms and at least one region of the down scalograms; or

the at least one characteristic comprises a peak in the original signal; or

the processor is further configured to select at least one ridge in at least one of the generated scalograms, wherein the at least one characteristic comprises consistency in at least one of the scale; or

the processor is further configured to normalize at least one of the scale and amplitude of the selected regions; or

the processor is further configured to sum the median amplitude for each scale increment in the concatenated scalogram.

12. The system of claim 8, wherein the processor is further configured to:

identify at least one outlier in the concatenated scalogram; and

apply a sum along amplitudes across time to regions of the concatenated scalogram that do not contain the at least one outlier.

13. The system of claim 8, wherein the processor is further configured to:

select a peak of the sum along amplitudes function; and

analyze the peak to obtain respiration information.

14. The system of claim 8, wherein the processor is further configured to:

identify a point of maximum curvature of the sum along amplitudes function; and

analyze the point to obtain respiration information.

**Patentansprüche**

1. Signalverarbeitungsverfahren, Folgendes umfassend:

Empfangen von Daten, die für ein ursprüngliches Signal an einem Sensor bezeichnend sind;
Erzeugen von Skalogrammen aus dem ursprünglichen Signal;
Auswählen von Bereichen der erzeugten Skalogramme, mindestens teilweise auf mindestens einer Charakteristik der erzeugten Skalogramme basierend; **gekennzeichnet durch**:

Konkatenieren der ausgewählten Bereiche, um ein konkateniertes Skalogramm zu formen;
Anwenden einer Summe entlang Amplituden über Zeit auf mindestens einen Teil des konkatenierten Skalogramms, um eine Funktion der Summe entlang Amplituden zu formen; und
Bestimmen eines gewünschten Parameters, mindestens teilweise auf der Funktion der Summe entlang Amplituden basierend.

2. Verfahren nach Anspruch 1, worin das Erzeugen von Skalogrammen das Erzeugen einer Vielzahl von Aufwärts-Skalogrammen und einer Vielzahl von Abwärts-Skalogrammen durch Folgendes umfasst:

Auswählen eines ersten Teils des ursprünglichen Signals;
Spiegeln des ersten Teils des ursprünglichen Signals an einer ersten vertikalen Achse, um einen gespiegelten ersten Teil zu erzeugen;
Auswählen eines nachfolgenden zweiten Teils des ursprünglichen Signals;
Spiegeln des zweiten Teils des ursprünglichen Signals an einer zweiten vertikalen Achse, um einen gespiegelten zweiten Teil zu erzeugen;
Kombinieren des gespiegelten ersten Teils und des gespiegelten zweiten Teils, um ein neues Signal zu erzeugen;
Transformieren des neuen Signals unter Verwendung einer Wavelet-Transformation; und
Erzeugen eines Skalogramms, mindestens teilweise auf dem transformierten Signal basierend, worin:

die ausgewählten Bereiche optional mindestens einen Bereich der Aufwärts-Skalogramme und mindestens einen Bereich der Abwärts-Skalogramme umfassen.

3. Verfahren nach Anspruch 1, worin das Erzeugen von Skalogrammen das Erzeugen einer Vielzahl von interpolierten Skalogrammen durch Folgendes umfasst:

Auswählen eines Teils des ursprünglichen Signals;
Erzeugen von Abtastwerten des ausgewählten Teils des ursprünglichen Signals unter Verwendung von mindestens einer Charakteristik des ursprünglichen Signals;
Interpolieren zwischen den Abtastwerten, um ein interpoliertes Signal zu erzeugen;
Transformieren des interpolierten Signals unter Verwendung einer Wavelet-Transformation; und
Erzeugen eines Skalogramms, mindestens teilweise auf dem transformierten Signal basierend, worin:

die mindestens eine Charakteristik optional eine Amplitude von mindestens einem eines Aufwärtstakts und eines Abwärtstakts eines Pulses im ursprünglichen Signal umfasst.

4. Verfahren nach Anspruch 1, worin:

die mindestens eine Charakteristik einen Spitzenwert im ursprünglichen Signal umfasst; oder
das Verfahren außerdem das Auswählen von mindestens einem Kamm in mindestens einem der erzeugten Skalogramme umfasst, worin die mindestens eine Charakteristik Konsistenz in mindestens einer der Skala und Amplitude von mindestens einem Kamm umfasst; oder
das Konkatenieren der ausgewählten Bereiche außerdem das Normalisieren von mindestens einer der Skala und Amplitude der ausgewählten Bereiche umfasst; oder
das Anwenden einer Summe entlang Amplituden über Zeit außerdem das Summieren der Median-Amplitude für jedes Skaleninkrement im konkatenierten Skalogramm umfasst.

5. Verfahren nach Anspruch 1, worin das Anwenden einer Summe entlang Amplituden über Zeit außerdem umfasst:

Identifizieren von mindestens einem Ausreißer im konkatenierten Skalogramm; und
Anwenden einer Summe entlang Amplituden über Zeit auf Bereiche des konkatenierten Skalogramms, die den mindestens einen Ausreißer nicht enthalten.

**6.** Verfahren nach Anspruch 1, worin das Bestimmen des gewünschten Parameters außerdem umfasst:

Auswählen eines Spitzenwerts der Funktion der Summe entlang Amplituden; und
Analysieren des Spitzenwerts, um Respirationsinformation zu erhalten.

**7.** Verfahren nach Anspruch 1, worin das Bestimmen des gewünschten Parameters außerdem umfasst:

Identifizieren eines Punktes maximaler Krümmung der Funktion der Summe entlang Amplituden; und
Analysieren des Punktes, um Respirationsinformation zu erhalten.

**8.** System zur Verarbeitung eines Signals, wobei das System umfasst:

einen Sensor zum Empfangen von Daten, die für ein ursprüngliches Signal bezeichnend sind;
einen an den Sensor gekoppelten Prozessor, worin der Prozessor konfiguriert ist zum:

Erzeugen von Skalogrammen aus dem ursprünglichen Signal;
Auswählen von Bereichen der erzeugten Skalogramme, mindestens teilweise auf mindestens einer Charakteristik der erzeugten Skalogramme basierend;
**dadurch gekennzeichnet, dass** der Prozessor außerdem konfiguriert ist zum:

Konkatenieren der ausgewählten Bereiche, um ein konkateniertes Skalogramm zu formen;
Anwenden einer Summe entlang Amplituden über Zeit auf mindestens einen Teil des konkatenierten Skalogramms, um eine Funktion der Summe entlang Amplituden zu formen; und
Bestimmen eines gewünschten Parameters, mindestens teilweise auf der Funktion der Summe entlang Amplituden basierend; und
einen an den Prozessor gekoppelten Ausgang, worin der Ausgang dazu konfiguriert ist, mindestens eins von Folgenden anzuzeigen: das konkatenierte Skalogramm, die Funktion der Summe entlang Amplituden und der bestimmte Parameter.

**9.** System nach Anspruch 8, worin der Prozessor außerdem konfiguriert ist zum:

Auswählen eines ersten Teils des ursprünglichen Signals;
Spiegeln des ersten Teils des ursprünglichen Signals an einer ersten vertikalen Achse, um einen gespiegelten ersten Teil zu erzeugen;
Auswählen eines nachfolgenden zweiten Teils des ursprünglichen Signals;
Spiegeln des zweiten Teils des ursprünglichen Signals an einer zweiten vertikalen Achse, um einen gespiegelten zweiten Teil zu erzeugen;
Kombinieren des gespiegelten ersten Teils und des gespiegelten zweiten Teils, um ein neues Signal zu erzeugen;
Transformieren des neuen Signals unter Verwendung einer Wavelet-Transformation; und
Erzeugen eines Skalogramms, mindestens teilweise auf dem transformierten Signal basierend.

**10.** System nach Anspruch 8, worin der Prozessor außerdem konfiguriert ist zum:

Auswählen eines Teils des ursprünglichen Signals;
Erzeugen von Abtastwerten des ausgewählten Teils des ursprünglichen Signals unter Verwendung von mindestens einer Charakteristik des ursprünglichen Signals;
Interpolieren zwischen den Abtastwerten, um ein interpoliertes Signal zu erzeugen;
Transformieren des interpolierten Signals unter Verwendung einer Wavelet-Transformation; und
Erzeugen eines Skalogramms, mindestens teilweise auf dem transformierten Signal basierend.

**11.** System nach Anspruch 8, worin:

die mindestens eine Charakteristik eine Amplitude von mindestens einem eines Aufwärtstakts und eines Abwärtstakts eines Pulses im ursprünglichen Signal umfasst; oder

die ausgewählten Bereiche mindestens einen Bereich der Aufwärts-Skalogramme und mindestens einen Bereich der Abwärts-Skalogramme umfassen; oder

die mindestens eine Charakteristik einen Spitzenwert im ursprünglichen Signal umfasst; oder

der Prozessor außerdem dazu konfiguriert ist, mindestens einen Kamm in mindestens einem der erzeugten Skalogramme auszuwählen, worin die mindestens eine Charakteristik Konsistenz in mindestens einer der Skala und Amplitude umfasst; oder

der Prozessor außerdem dazu konfiguriert ist, mindestens eine der Skala und Amplitude der ausgewählten Bereiche zu normalisieren; oder

der Prozessor außerdem dazu konfiguriert ist, die Median-Amplitude für jedes Skaleninkrement im konkatenierten Skalogramm zu summieren.

**12.** System nach Anspruch 8, worin der Prozessor außerdem konfiguriert ist zum:

Identifizieren von mindestens einem Ausreißer im konkatenierten Skalogramm; und
Anwenden einer Summe entlang Amplituden über Zeit auf Bereiche des konkatenierten Skalogramms, die nicht den mindestens einen Ausreißer enthalten.

**13.** System nach Anspruch 8, worin der Prozessor außerdem konfiguriert ist zum:

Auswählen eines Spitzenwerts der Funktion der Summe entlang Amplituden; und
Analysieren des Spitzenwerts, um Respirationsinformation zu erhalten.

**14.** System nach Anspruch 8, worin der Prozessor außerdem konfiguriert ist zum:

Identifizieren eines Punktes maximaler Krümmung der Funktion der Summe entlang Amplituden; und
Analysieren des Punktes, um Respirationsinformation zu erhalten.

**Revendications**

**1.** Procédé de traitement de signal, consistant à :

recevoir des données indicatives d'un signal initial au niveau d'un capteur ;
générer des scalogrammes à partir du signal initial ;
sélectionner des zones des scalogrammes générés, sur la base, au moins en partie, d'au moins une caractéristique des scalogrammes générés, **caractérisé en ce qu'**il consiste à :

enchaîner les zones sélectionnées en vue de former un scalogramme enchaîné ;
appliquer une somme le long des amplitudes dans le temps à au moins une partie du scalogramme enchaîné, en vue de former une fonction de somme le long des amplitudes ; et
déterminer un paramètre souhaité, sur la base, au moins en partie, de la fonction de somme le long des amplitudes.

**2.** Procédé selon la revendication 1, dans lequel la génération de scalogrammes consiste à générer une pluralité de scalogrammes ascendants et une pluralité de scalogrammes descendants :

en sélectionnant une première partie du signal initial ;
en reflétant la première partie du signal initial sur un premier axe vertical, en vue de créer une première partie réfléchie ;
en sélectionnant une seconde partie subséquente du signal initial ;
en reflétant la seconde partie du signal initial sur un second axe vertical, en vue de créer une seconde partie réfléchie ;
en combinant la première partie réfléchie et la seconde partie réfléchie, en vue de créer un nouveau signal ;
en transformant le nouveau signal au moyen d'une transformée en ondelettes ; et
en générant un scalogramme sur la base, au moins en partie, du signal transformé ;
dans lequel :

facultativement, les zones sélectionnées comprennent au moins une zone des scalogrammes ascendants et au moins une zone des scalogrammes descendants.

3. Procédé selon la revendication 1, dans lequel la génération de scalogrammes consiste à générer une pluralité de scalogrammes interpolés :

en sélectionnant une partie du signal initial ;
en générant des échantillons de la partie sélectionnée du signal initial en utilisant au moins une caractéristique du signal initial ;
en mettant en oeuvre une interpolation entre les échantillons, en vue de créer un signal interpolé ;
en transformant le signal interpolé au moyen d'une transformée en ondelettes ; et
en générant un scalogramme sur la base, au moins en partie, du signal transformé ;
dans lequel :

facultativement, ladite au moins une caractéristique comprend une amplitude d'au moins l'une parmi une course ascendante et une course descendante d'une impulsion dans le signal initial.

4. Procédé selon la revendication 1, dans lequel :

ladite au moins une caractéristique comprend une crête dans le signal initial ; ou
le procédé consiste en outre à sélectionner au moins une arête dans au moins l'un des scalogrammes générés, dans lequel ladite au moins une caractéristique comprend une cohérence dans au moins l'une de l'échelle et l'amplitude d'au moins une arête ; ou
l'enchaînement des zones sélectionnées consiste en outre à normaliser au moins l'une de l'échelle et l'amplitude des zones sélectionnées ; ou
l'application d'une somme le long des amplitudes dans le temps consiste en outre à sommer l'amplitude médiane pour chaque incrément d'échelle dans le scalogramme enchaîné.

5. Procédé selon la revendication 1, dans lequel l'application d'une somme le long des amplitudes dans le temps consiste en outre à :

identifier au moins une aberration dans le scalogramme enchaîné ; et
appliquer une somme le long des amplitudes dans le temps à des zones du scalogramme enchaîné qui ne contiennent pas ladite au moins une aberration.

6. Procédé selon la revendication 1, dans lequel la détermination du paramètre souhaité consiste en outre à :

sélectionner une crête de la fonction de somme le long des amplitudes ; et
analyser la crête en vue d'obtenir des informations de respiration.

7. Procédé selon la revendication 1, dans lequel la détermination du paramètre souhaité consiste en outre à :

identifier un point de courbure maximale de la fonction de somme le long des amplitudes ; et
analyser le point en vue d'obtenir des informations de respiration.

8. Système de traitement d'un signal, le système comprenant:

un capteur destiné à recevoir des données indicatives d'un signal initial ;
un processeur couplé au capteur, dans lequel le processeur est configuré de manière à :

générer des scalogrammes à partir du signal initial ;
sélectionner des zones des scalogrammes générés sur la base, au moins en partie, d'au moins une caractéristique des scalogrammes générés,
**caractérisé en ce que** le processeur est en outre configuré de manière à :

enchaîner les zones sélectionnées en vue de former un scalogramme enchaîné ;
appliquer une somme le long des amplitudes dans le temps à au moins une partie du scalogramme enchaîné, en vue de former une fonction de somme le long des amplitudes ; et

déterminer un paramètre souhaité sur la base, au moins en partie, de la fonction de somme le long des amplitudes ; et

une sortie couplée au processeur, dans laquelle la sortie est configurée de manière à afficher au moins l'un des éléments parmi le scalogramme enchaîné, la fonction de somme le long des amplitudes, et le paramètre déterminé.

9. Système selon la revendication 8, dans lequel le processeur est en outre configuré de manière à :

sélectionner une première partie du signal initial ;
refléter la première partie du signal initial sur un premier axe vertical en vue de créer une première partie réfléchie ;
sélectionner une seconde partie subséquente du signal initial ;
refléter la seconde partie du signal initial sur un second axe vertical en vue de créer une seconde partie réfléchie ;
combiner la première partie réfléchie et la seconde partie réfléchie en vue de créer un nouveau signal ;
transformer le nouveau signal au moyen d'une transformée en ondelettes ; et
générer un scalogramme sur la base, au moins en partie, du signal transformé.

10. Système selon la revendication 8, dans lequel le processeur est en outre configuré de manière à :

sélectionner une partie du signal initial ;
générer des échantillons de la partie sélectionnée du signal initial en utilisant au moins une caractéristique du signal initial ;
mettre en oeuvre une interpolation entre les échantillons, en vue de créer un signal interpolé ;
transformer le signal interpolé au moyen d'une transformée en ondelettes ; et
générer un scalogramme sur la base, au moins en partie, du signal transformé.

11. Système selon la revendication 8, dans lequel :

ladite au moins une caractéristique comprend une amplitude d'au moins l'une parmi une course ascendante et une course descendante d'une impulsion dans le signal initial ; ou
les zones sélectionnées comprennent au moins une zone des scalogrammes ascendants et au moins une zone des scalogrammes descendants ; ou
ladite au moins une caractéristique comprend une crête dans le signal initial ; ou
le processeur est en outre configuré de manière à sélectionner au moins une arête dans au moins l'un des scalogrammes générés, dans laquelle ladite au moins une caractéristique comprend une cohérence dans au moins l'une parmi l'échelle et l'amplitude ; ou
le processeur est en outre configuré de manière à normaliser au moins l'une parmi l'échelle et l'amplitude des zones sélectionnées ; ou
le processeur est en outre configuré de manière à sommer l'amplitude médiane pour chaque incrément d'échelle dans le scalogramme enchaîné.

12. Système selon la revendication 8, dans lequel le processeur est en outre configuré de manière à :

identifier au moins une aberration dans le scalogramme enchaîné ; et
appliquer une somme le long des amplitudes dans le temps à des zones du scalogramme enchaîné qui ne contiennent pas ladite au moins une aberration.

13. Système selon la revendication 8, dans lequel le processeur est en outre configuré de manière à :

sélectionner une crête de la fonction de somme le long des amplitudes ; et
analyser la crête en vue d'obtenir des informations de respiration.

14. Système selon la revendication 8, dans lequel le processeur est en outre configuré de manière à :

identifier un point de courbure maximale de la fonction de somme le long des amplitudes ; et
analyser le point en vue d'obtenir des informations de respiration.

FIG.1

FIG. 2

EP 2 303 110 B1

FIG. 3(a)

FIG. 3(b)

EP 2 303 110 B1

FIG. 3(c)

FIG. 3(d)

Wavelet transform (WT)                                    Original signal

Take first time, *t*

Take first scale, *a*

Calculate dot product of
current WT scale, *a*, with
wavelet of scale *a* centred
at time *t*.

Get next
scale, *a*

Factor by *db*. Hold
result for later use.

Go to next
time, *t*

No

All scales
done?

No

All time
points done?

Yes

Yes

Find weighted sum of held
data across all scales to find
original signal at time, *t*

*inverse continuous wavelet transform*

FIG. 3(e)

Wavelet transform          Original signal

```
Take first scale

Calculate Fourier
Transform of scale

Multiply by scaled Fourier
Transform of wavelet at
that scale

Calculate inverse
Fourier transform and
Hold result for later
use

Get next scale

All scales
done?

No          Yes

Take real value
of resultant
summation

Find weighted sum of
held data for each
time across all scales
```

*Fast inverse wavelet transform*

## FIG. 3(f)

FIG. 4

EP 2 303 110 B1

## 500

Start — 502

↓

Select a first portion of a signal — 504

↓

Mirror first portion about a first vertical axis to create mirrored first portion — 506

↓

Select a second portion of the signal — 508

↓

Mirror second portion about a second vertical axis to create mirrored second portion — 510

↓

Combine mirrored first portion and mirrored second portion to create a new signal — 512

↓

Analyze the new signal — 514

↓

End — 516

## FIG. 5

6400

6410

t

6405

B

6420

U

D

6430

U

D

6440

6443

6445

6450

6453

6455

6460

t

6463

6465

# FIG. 6

<u>700</u>

Start — 702

↓

| Select a portion of a signal | — 704 |

↓

| Sample signal to obtain characteristic points of the signal | — 706 |

↓

| Interpolate between the samples to create a new signal | — 708 |

↓

| Analyze the new signal | — 710 |

↓

End — 712

# FIG. 7

8400

FIG. 8

**900**

```
┌─────────────────────────────────┐  910
│   Receive data from sensor to form  │
│          original signal          │
└─────────────────────────────────┘
                 │
                 ▼
┌─────────────────────────────────┐  920
│   Generate signals and / or scalograms  │
│         from original signal       │
└─────────────────────────────────┘
                 │
                 ▼
┌─────────────────────────────────┐  930
│      Select regions of scalograms    │
└─────────────────────────────────┘
                 │
                 ▼
┌─────────────────────────────────┐  940
│      Concatenate selected regions    │
└─────────────────────────────────┘
                 │
                 ▼
┌─────────────────────────────────┐  950
│      Analyze concatenated scalogram   │
└─────────────────────────────────┘
```

FIG. 9

<u>1000</u>

1030

Generate Up/Down
Signals

Up Signal
1033

Down Signal
1035

Generate Up
Scalogram 1043

Generate Down
Scalogram 1045

1040

Extract Up
Ridge 1053

Extract Down
Ridge 1055

1050

1060

Generate
Secondary Up
Scalogram 1063

Generate
Secondary Down
Scalogram 1065

Generate Selected
Scalograms 1067

Select Regions of
Scalograms

1070

1075

Construct Concatenated
Scalogram 1077 Using Selected
Regions

1080

Sum Amplitudes Across Time
for Each Scale in Concatenated Scalogram 1077

1090

Determine Respiration Rate

FIG. 10

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61077062 B
- US 61077130 B
- US 61077092 A, Watson
- US 43732609 A, Watson

**Non-patent literature cited in the description**

- **PAUL S. ADDISON.** The Illustrated Wavelet Transform Handbook. Taylor & Francis Group, 2002